# EUROPEAN PATENT SPECIFICATION

(11) **EP 3 851 060 B1**
(45) Date of publication and mention of the grant of the patent: **09.08.2023**
(21) Application number: 19861079.2
(22) Date of filing: 12.04.2019
(51) Int. Cl.: A61B 18/14, A61N 5/00

(54) **RADIO FREQUENCY ABLATION CATHETER, RADIO FREQUENCY ABLATION SYSTEM FOR LUNGS**
RADIOFREQUENZABLATIONSKATHETER, RADIOFREQUENZABLATIONSSYSTEM FÜR LUNGEN
CATHÉTER D'ABLATION PAR RADIOFRÉQUENCE, SYSTÈME D'ABLATION PAR RADIOFRÉQUENCE POUR LES POUMONS

(30) Priority: 14.09.2018 CN 201811075817; 14.09.2018 CN 201811076707
(43) Date of publication of application: 21.07.2021
(73) Proprietor: Hangzhou Broncus Medical Co., Ltd., Hangzhou, Zhejiang 310051 (CN)
(72) Inventor: XU, Hong, Hangzhou, Zhejiang 310051 (CN); ZHOU, Huazhen, Hangzhou, Zhejiang 310051 (CN); WANG, Liming, Hangzhou, Zhejiang 310051 (CN); JIANG, Song, Hangzhou, Zhejiang 310051 (CN); SU, Chenhui, Hangzhou, Zhejiang 310051 (CN)
(74) Representative: MERH-IP Matias Erny Reichl Hoffmann Patentanwälte PartG mbB
(86) International application number: PCT/CN2019/082546
(87) International publication number: WO 2020/052231

(56) References cited:
- WO-A1-2015/089377
- WO-A1-2016/081650
- CN-A- 1 631 327
- CN-A- 1 777 396
- CN-A- 1 897 885
- CN-A- 105 997 234
- CN-A- 106 073 891
- CN-U- 202 892 082
- US-A1- 2003 004 506
- US-A1- 2003 004 506
- US-A1- 2010 016 848
- US-A1- 2017 209 203

## Description

### TECHNICAL FIELD

This application relates to the field of minimally invasive ablation therapy for tumors, and in particular to a radio frequency ablation catheter, and a radio frequency ablation system and method for lungs.

### BACKGROUND

Lung cancer is one of the most common malignant tumors. In clinical treatment, surgical resection is still the first choice for the treatment of early lung cancer. However, patients with lung cancer who are older and weaker, have poor cardiopulmonary function, or have complications, are not suitable or cannot tolerate the conventional surgical resection. Therefore, many local treatments such as minimally invasive tumor ablation have emerged as the times require. Minimally invasive ablation of lung tumors includes radio frequency ablation (RFA), cryoablation, microwave ablation, etc. Among them, only radio frequency ablation is incorporated in the National Comprehensive Cancer Network Clinical practice guidelines in oncology: Non-small cell lung cancer.

The principle of radio frequency ablation is to apply a high-frequency alternating current with a frequency of less than 30 MHz (usually 460-480 kHz) to cause ions in the tumor tissue to oscillate at a high speed and rub each other, and the radio frequency energy is converted into heat energy, so that the tumor cells undergo coagulative necrosis. In the radio frequency ablation treatment, the instrument used is a radio frequency ablation catheter, having an electrode at a distal end that can transmit radio frequency energy to the cells and tissues around a punctured site after percutaneous puncture. During the radio frequency ablation treatment, the radio frequency ablation catheter is an electrode for outputting the radio frequency energy, which is connected to a radio frequency generator, and is guided by B-ultrasound or CT to percutaneously puncture into a target tumor through a puncture point. A neutral electrode pad is further connected to the radio frequency generator, and attached to a suitable site of the patient's body. When a foot switch of the radio frequency generator is pressed down, the radio frequency ablation catheter and the neutral electrode pad are electrically connected and a high-frequency current acts on the human tissue therebetween, causing the coagulation, denaturation, and necrosis of tumor cells in contact with the electrode at the distal end of the radio frequency ablation catheter.

The present inventors find that when the traditional radio frequency ablation catheter for lungs is in operation, the temperature of the electrode portion increases too fast, and "scabs" are formed after the tissue near the electrode is dried and charred, causing the ablation to stop and thus resulting in incomplete ablation. Moreover, the "scab" tissue adheres to the electrode, causing damage to the surrounding organs when the apparatus is withdrawn. United States Patent Application No. US20030004506A1 discloses typical catheters with irrigated tip electrodes for reducing the heating of the tip resulting from RF biasing.

The heads of most of the traditional radio frequency ablation catheters for lungs cannot be bent, such that the distal electrode of the radio frequency ablation catheter cannot conveniently reach a target position at a lateral side.

For the traditional radio frequency catheters, it is unable to effectively control the range of ablation and determine whether the range of ablation is appropriate in time. If the range of ablation is smaller, the ablation is incomplete, and there is a risk of recurrence; and if the range of ablation is larger, the surrounding normal tissues and organs may be unintentionally injured.

Even under the guidance of B-ultrasound or CT, the traditional radio frequency ablation operations cannot effectively determine the accurate position of the distal electrode of the radio frequency ablation catheter. A CT image consists of a limited number of cross-sectional images scanned by X-rays. At certain angles, it seems as if the distal electrode is positioned in a target position, but the actual position of the distal electrode may be wrong, because they may just overlap in the projection direction. Therefore, the position of the distal electrode is difficult to be determined, and the positioning accuracy is insufficient.

### SUMMARY

To solve at least part of the problems mentioned in the background, an objective of this application is to provide a radio frequency ablation catheter according to claim 1, having an electrode allowing a heat exchange medium to be perfused into a tissue to be ablated, and forming a heat exchange medium protective layer formed on the outer periphery of the electrode, thereby improving the electrical and thermal conductivity of the tissue to be ablated, maintaining balance of the impedance, thus maintaining the impedance in a relatively stable state, and enabling continuous output of radio frequency energy.

The invention is defined in the appended claims. Aspects, embodiments and examples disclosed herein which do not fall within the scope of the appended claims do not form part of the invention and are merely provided for illustrative purposes. Furthermore, the methods presented in the present description are not claimed as such but are useful for understanding of the claimed subject matter, in particular since the methods can be performed by the claimed subject-matter.

### BRIEF DESCRIPTION OF THE DRAWINGS

FIG. 1 is a view showing the overall structure of a radio frequency ablation catheter;
FIG. 2 is a cross-sectional view of an insertion portion;
FIG. 3 is another cross-sectional view of the insertion portion;
FIG. 4 is a cross-sectional view of a handle portion;
FIG. 5 is another cross-sectional view of the handle portion;
FIG. 6a is a view showing the external structure of a first stretch bending component;
FIG. 6b is a cross-sectional view along A-A in FIG. 6a;
FIG. 7a is a view showing the external structure of a second stretch bending component;
FIG. 7b is a cross-sectional view along A-A in FIG. 7a;
FIG. 8 is a structural view showing an electrode, wherein an infiltration cover and the electrode are formed as one-piece structure;
FIG. 9 is a structural view showing an electrode, wherein an infiltration cover and the electrode are separately formed;
FIG. 10a is a cross-sectional view of FIG. 9;
FIG. 10b is a partial cross-sectional view of the electrode;
FIG. 10c is a partial cross-sectional view of the electrode;
FIG. 10d is a partial cross-sectional view of the electrode;
FIG. 11 is a view showing the overall structure of a radio frequency ablation catheter;
FIG. 12 is a partially enlarged view of FIG. 11;
FIG. 13 is a cross-sectional view of FIG. 11;
FIG. 14 is a structural view showing an electrode, wherein an infiltration cover and the electrode are formed as one-piece structure;
FIG. 15 is a view of the electrode in FIG. 14 viewed from another aspect;
FIG. 16 is a cross-sectional view along A-A in FIG. 15;
FIG. 17 is a view of the electrode in FIG. 14 viewed from another aspect;
FIG. 18a is a schematic view showing the change of the temperature around an electrode during ablation;
FIG. 18b is a schematic view showing an installation location of a temperature detection device in a radio frequency ablation catheter;
FIG. 18c is a schematic view, wherein a heat conducting ring in FIG. 18b is omitted;
FIG. 18d is a cross-sectional view at a position of the heat conducting ring;
FIG. 19a is a flow chart of a control method for radio frequency ablation;
FIG. 19b is a schematic view showing the temperature distribution in the lesion site during radio frequency ablation;
FIG. 20 is a schematic diagram showing a hardware structure in computer equipment;
FIG. 21 is a flow chart of a control method for radio frequency ablation for lungs;
FIG. 22 is a flow chart of a control method for radio frequency ablation for lungs;
FIG. 23 is a schematic diagram of a radio frequency ablation system for lungs;
FIG. 24 is a schematic diagram of another radio frequency ablation system for lungs;
FIG. 25 is a schematic diagram of control method for radio frequency ablation for lungs;
FIG. 26 is a schematic diagram showing another control method for radio frequency ablation for lungs.

### List of reference numerals:

1. electrode; 101. saline connecting tube; 102. first mounting hole; 103. second mounting hole; 104. third mounting hole; 105. fourth mounting hole; 106. saline hole; 107. recessed area; 111. connecting tube; 112. mounting hole; 113. mounting hole; 114. tip; 115. main flow channel; 116. branch flow channel; 117. branch flow channel; 118. branch flow channel; 119. dispersing hole; 2. sheath; 3. protective tube; 4. first stretch bending component; 400. ring; 401. sliding chamber; 402. connecting joint; 403. limit screw; 5. second stretch bending component; 500. wire running cavity; 501. sliding tube body; 502. sliding groove; 503. bolt hole; 504. groove; 6. saline joint; 7. ablation apparatus connector; 8. electrode ring; 9. thermistor; 90. thermistor wire; 91. temperature control sleeve; 10. pull wire; 11. spring hose; 12. saline tube; 13. pull-wire fixing bolt; 14. end cover; 15. O-ring; 16. lead wire; 17. pressure sensor; 18. bifurcated riveting tube; 19. temperature sensor; 20. infiltration cover; 200. dispersing hole; 201. dispersing hole; 202. dispersing hole; 21. metal tube; 22. temperature sensor; 23. heat exchange medium delivery tube; 24. electrode wire; 25. Y-shaped handle; 26. handle cover; 27. connector; 28. Luer joint; 29. temperature sensor; 211. radio frequency ablation catheter; 212. engaging groove; 213. first through hole; 214. heat conducting ring; 215. recessed groove; 216. second through hole; 100. radio frequency signal generator; 110. ablation catheter; 120. sensor module; 130. microperfusion pump; 140. control module; 150. alarm module.

### DESCRIPTION OF THE EMBODIMENTS

The technical solutions in the embodiments of the present application will be described clearly and fully with reference to the accompanying drawings thereof. Apparently, the embodiments described are merely some, rather than all of the embodiments of the present application. All other embodiments obtained by a person of ordinary skill in the art without creative efforts based on the embodiments of the present application shall fall within the protection scope of the present application.

For better description and illustration of the embodiments of this application, one or more drawings may be referred to; however, the additional details or examples used to describe the drawings should not be regarded as limitations to the scopes of any one of the inventions and creations, embodiments or preferred implementations described here of this application.

It should be noted that when a component is described to be "connected" to another component, it can be directly connected to the other component or there may be an intermediate component. When a component is considered to be "provided on" another component, it can be directly provided on the other component or there may be an intermediate component.

Unless otherwise defined, all technical and scientific terms used herein have the same meaning as commonly understood by a person skilled in the art to which the present application pertains. The terms used in the description of the present application are for the purpose of illustrating particular embodiments only and are not intended to limit the present application. The term "and/or" as used herein includes any and all combinations of one or more of the listed related items.

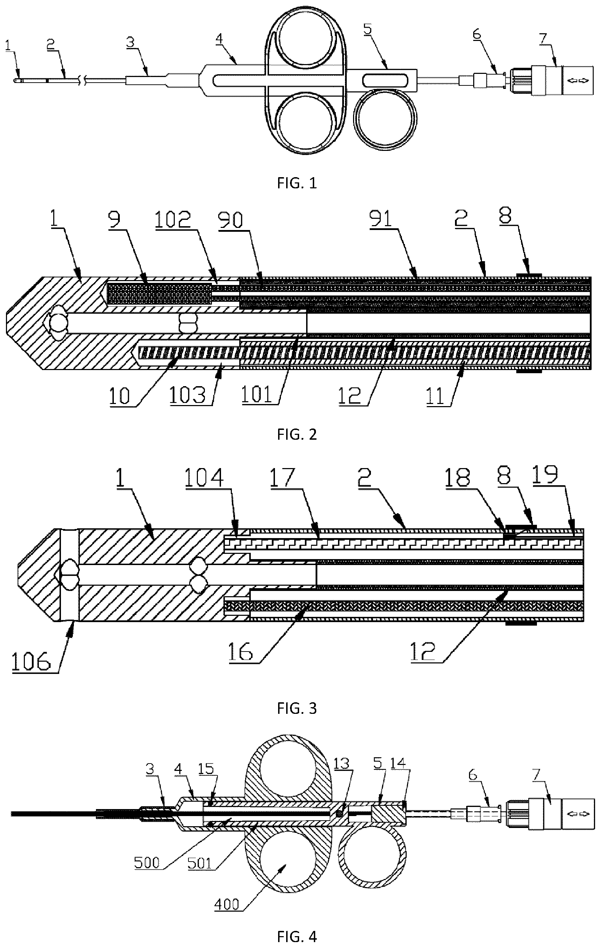

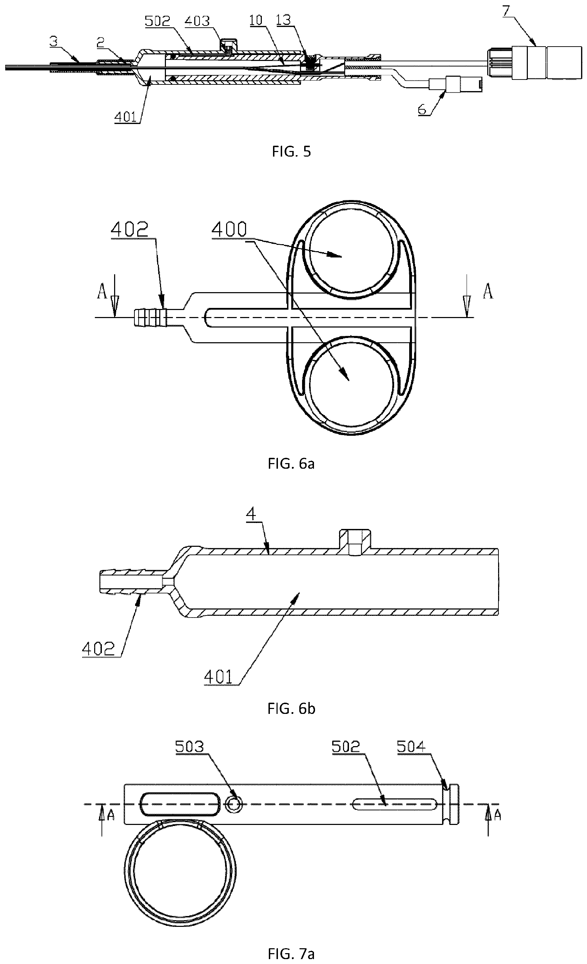

As shown in FIGs. 1 to 18a, a radio frequency ablation catheter generally includes an insertion portion and a handle portion, wherein a distal end of the insertion portion is provided with an electrode 1.

The radio frequency ablation catheter according to an embodiment of the present application includes an electrode 1. A heat exchange medium flow channel is defined in the electrode 1. A dispersing device is provided on the electrode 1. At least one dispersing hole is provided in the dispersing device which is communicating with the heat exchange medium flow channel. A heat exchange medium output from the heat exchange medium flow channel is distributed by and flows out of the dispersing device.

In this embodiment, the heat exchange medium output from the heat exchange medium flow channel is further distributed by the dispersing device to form a heat exchange medium protective layer between the electrode 1 and the lesion tissue. The concept and principle of this design are different from those of the common cooling means. The heat exchange medium can be distributed by the manner of multiple holes or a slit, etc., to at least avoid outputting the heat exchange medium at a same site. By virtue of the distribution, a relatively uniform heat exchange medium protective layer is formed on the outer surface of the electrode 1, which can reduce the temperature and increase the moisture content of the tissue, thus fundamentally preventing the tissue from scabbing due to drying and heating. Moreover, the heat exchange medium can improve the electrical and thermal conductivity of the tissue, maintain balance of the impedance, and achieve stable progression of the ablation.

In one of the embodiments, the dispersing device and the electrode are separately formed, and the dispersing device is fixedly or movably mounted on the electrode. Alternatively, the dispersing device and the electrode are formed as one-piece structure.

When the dispersing device and the electrode are formed as one-piece structure, that is, the dispersing device is a part of the electrode, the dispersing hole is defined at an outer wall of the electrode. The dispersing device surrounds at least partial area of the heat exchange medium flow channel, and the dispersing hole on the dispersing device corresponds to, that is, communicates with, the area. The dispersing device may be a section of the electrode in the axial direction, and the section is preferably continuously arranged in the circumferential direction. There is a plurality of dispersing holes distributed in the circumferential direction, so that the distribution of the heat exchange medium is much better, and the heat exchange medium protective layer formed is more even, thus avoiding that the protective layer is only formed partially in the circumferential direction.

In one of the embodiments, multiple dispersing holes are provided for forming a uniform heat exchange medium protective layer on the outside of the electrode.

Multiple dispersing holes are more advantageous to the uniform distribution of the heat exchange medium. The dispersing holes can be arranged in a regular manner or pattern on the outer periphery of the dispersing device, or they can be randomly distributed. The heat exchange medium output from the heat exchange medium flow channel permeates and flows out to the outside of the dispersing device through each dispersing hole, and then surrounds the electrode to form a uniform heat exchange medium protective layer. Specific distribution of the dispersing hole is provided in further preferred embodiments hereinafter.

The diameter of the dispersing hole is ranged from 0.1 to 0.3 mm. A suitable diameter is more advantageous to the distribution and formation of the heat exchange medium protective layer. In case that the dispersing hole is non-circular, the size of which can be converted with reference to the area of a round hole to ensure the flow rate of the heat exchange medium at the dispersing hole.

In one of the embodiments, the dispersing hole is slit-shaped. Compared with a common shape, the slit shape has an obvious lengthwise direction, for example, the length is over 5 times the width. The width of the slit is generally about 0.1 mm. The lengthwise direction of the slit extends along the axial or circumferential direction of the electrode, or forms an angle with the axial direction.

In a radio frequency ablation catheter according to an implementation of the present application, the dispersing device and the electrode are separately formed, an outflow hole communicating with the heat exchange medium flow channel is defined on an outer wall of the electrode 1, the dispersing device is an infiltration cover 20 mounted on the electrode 1 surrounding the outflow hole, and the dispersing hole is defined on the infiltration cover 20, wherein the heat exchange medium output from the outflow hole is distributed by and flows out of the infiltration cover 20.

In this application, the distal end is understood as the end adjacent to the lesion site; the proximal end is understood as the end far from the lesion site, that is, the end adjacent to the handle portion; and the axial direction is understood as the extension direction of the sheath. Although the sheath can be partially bent, it can be understood with reference to its state before bending.

As for the electrode itself, except for the most distal portion, the rest portion is generally a revolving body structure, such as a cylindrical shaped structure, so it has spatially axial and radial directions, and the axial direction of the electrode is the same as the extension direction of the sheath.

In a preferred embodiment, all the outflow holes are covered by the infiltration cover 20, but in some embodiments, a portion of the outflow holes may be exposed from the infiltration cover 20, that is, not covered by the infiltration cover 20.

In one of the embodiments, a distal portion of the electrode is equal-diameter extended or converged in shape, wherein the converged shape may be gradually converged or stepwise converged.

The converged shape facilitates the implementation of the puncture in the body. When converged, the diameter or outer contour gradually becomes smaller toward the distal end, and the trend of becoming smaller can be fixed (i.e., gradually converged) or variable (i.e., stepwise converged).

In one of the embodiments, the distal portion of the electrode is a cylinder, a spherical cap, a truncated cone, a truncated pyramid, a cone, a pyramid or a structure obtained by inclinedly cutting the structures of above-mentioned shape (i.e., the cylinder, the spherical cap, the truncated cone, the truncated pyramid, the cone or the pyramid) with at least one face.

Inclinedly cutting the structures with one or more faces allows for more variations in the shape of the distal end of the electrode. Oblique cutting means that the inclinedly cut face is not parallel or perpendicular to the axis of the electrode. Oblique cutting is impossible in parallel arrangement. Perpendicular arrangement means that an end surface of the distal end of the electrode is flat, such as that in a cylindrical or truncated cone structure.

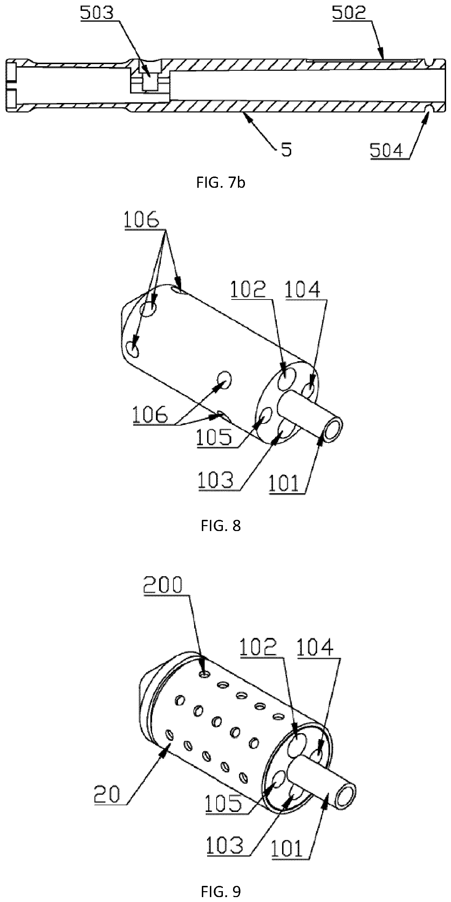

For example, the distal end of electrode 1 in FIG. 9 is in a truncated cone shape.

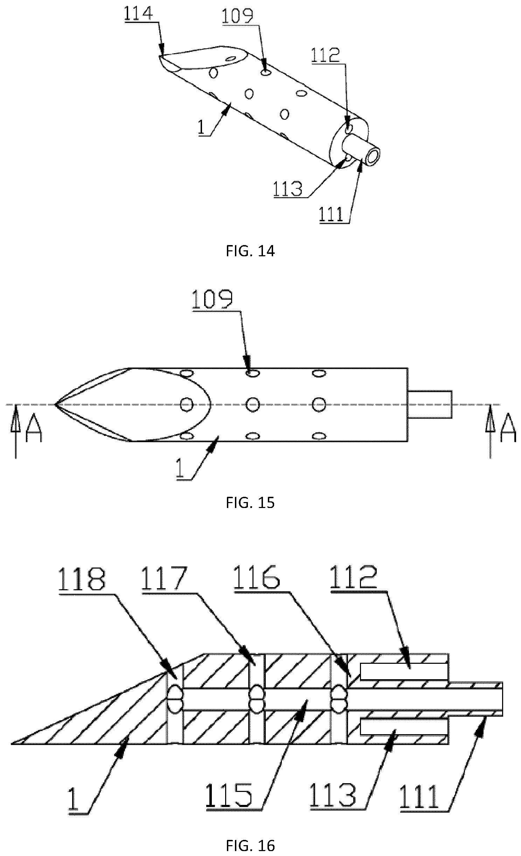

In one of the embodiments, the dispersing device and the electrode are formed as one-piece structure, the dispersing hole is defined on an outer wall of the electrode, and the distal end of the electrode is cuspidal. For example, the distal end of the electrode 1 in FIG. 14 is a tip 114, which specifically can be deemed as a structure formed by inclinedly cutting a cylinder with three faces. The tip 114 facilitates implement the puncture in the body.

Although some preferred embodiments are provided above, the shape of the distal end of the electrode itself may be implemented as those known in the prior art.

In this embodiment, the heat exchange medium flow channel may be communicated with an external heat exchange medium delivery device through a connecting tube, and necessary pump, valve, or metering device may be provided on the pipeline as required. For example, in FIG. 16, a proximal end of the electrode 1 is provided with a connecting tube 111, and a main flow channel 115 inside the electrode 1 is connected to and communicated with the connecting tube 111 to transfer the heat exchange medium.

As for the heat exchange medium itself, those known in the prior art can be used. It is mainly for cooling in the ablation and cooling surrounding sites. However, in some cases, it may also be used for heating, the relationship between the temperature of the heat exchange medium and body temperature may be adjusted according to the need.

Since the heat exchange medium is required to be delivered into the body, a material that is pharmaceutically acceptable for the human body is used. With respect to its physical form, it can be in the form of a gas, a liquid or a solid powder with a fluidity, or a combination of multiple physical forms. The heat exchange medium may be a pure substance or a mixture, and may be in combination with medicine administration.

In one of the embodiments, the heat exchange medium flow channel is a lumen inside the electrode 1, and the heat exchange medium flows out through an opening in the wall surrounding the lumen.

The lumen may be of a cylinder shape, a sphere shape, a spherical cap shape or a combination of the above shapes, etc. A partial outer periphery of the lumen is close to the outer surface of the electrode, that is, the wall surrounding the lumen corresponding to this area is thin, which is convenient for form openings. The position of the lumen in the electrode is adapted to the distributed area of the openings. In this way, the lumen can not only be used to deliver the heat exchange medium, but also pre-distribute or mix the heat exchange medium, to ensure that the temperature and concentration of the heat exchange medium output through different openings are consistent. Especially when the heat exchange medium is supplied in combination with a medicine administration, the effect is more prominent.

In case that the dispersing device and the electrode are formed as one-piece structure, the opening serves as the dispersing hole. In case that the dispersing device and the electrode are separately formed, the opening can be deemed as the outflow hole which is defined on the outer wall of the electrode.

In one of the embodiments, the heat exchange medium flow channel includes at least one main flow channel and a plurality of branch flow channels communicating with the main flow channel, wherein the end of each branch flow channel extends to the outer surface of the electrode.

The at least one main flow channel may include one main flow channel or a plurality of main flow channels arranged side-by-side, and preferably one main flow channel is provided at the axis of the electrode and extends along the axial direction of the electrode. Each branch flow channel communicates with the main flow channel at the same position or different positions.

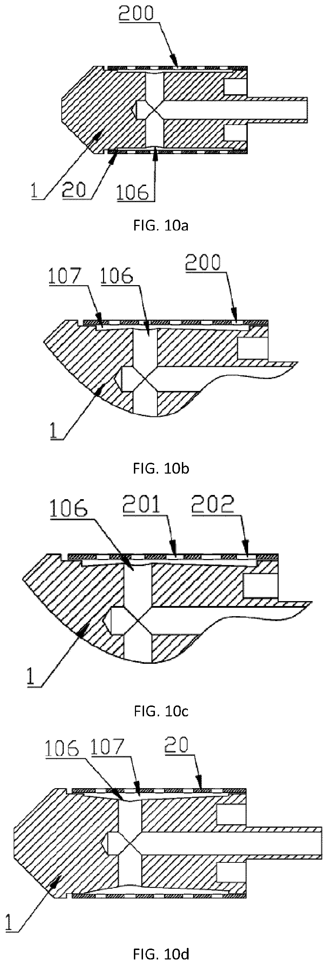

For example, FIG. 10a shows a main flow channel extending along the axial direction of the electrode, and multiple branch flow channels communicate with the main flow channel at the same position.

FIG. 16 shows a main flow channel 115 extending along the axial direction of the electrode, and multiple branch flow channels communicate with the main flow channel 115 at different positions.

In case that the dispersing device and the electrode are formed as one-piece structure, the end of each branch flow channel extends to the dispersing hole on the outer surface of the electrode 1. In case that the dispersing device and the electrode are separately formed, the end of each branch flow channel extends to the outflow hole on the outer surface of the electrode 1.

In one of the embodiments, at least one group of branch flow channels are arranged along the extension direction of the main flow channel, and at least two branch flow channels are included in the same group, which are radially distributed on the outer periphery of the main flow channel.

In order to facilitate processing, the extension direction of the branch flow channel is preferably the radial direction of the electrode. Of course, the branch flow channel may be arranged inclinedly to the radial direction. The inclination angles of the branch flow channels can be the same or different. The axial position of each branch flow channel is considered based on the position communicating with the main flow channel.

For example, as shown in FIG. 10a, one group of branch flow channels are arranged in the extension direction of the main flow channel, and in FIG. 16 three groups of branch flow channels are arranged along the extension direction of the main flow channel. As can be seen, a branch flow channel 116, a branch flow channel 117, and a branch flow channel 118 communicate with the main flow channel 115 at different positions (in the axial direction). Branch flow channels of the same group are distributed radially.

In one of the embodiments, the branch flow channels of the same group are evenly arranged in the circumferential direction.

When distributed radially, a circumferentially uniform arrangement may obtain a relatively uniform outflow.

In one of the embodiments, the number of branch flow channels in adjacent groups may be the same or different, and their positions are aligned or offset in the circumferential direction.

For example, as shown in FIG. 16, the numbers of branch flow channels in adjacent groups are the same, and the positions of them in the circumferential direction are aligned. That is, the branch flow channel 116, the branch flow channel 117, and the branch flow channel 118 in this figure are aligned in the circumferential direction. Of course, in other embodiments, the branch flow channel 116, the branch flow channel 117 and the branch flow channel 118 may be arranged in an offset manner in the circumferential direction.

In one of the embodiments, a plurality of branch flow channels are arranged in sequence along the extension direction of the main flow channel, in a spiral manner on the outer periphery of the main flow channel. The most commonly used heat exchange medium is physiological saline. For convenience of description and understanding, physiological saline is taken as an example in some embodiments below. Accordingly, the outflow hole may be referred to as saline hole, the heat exchange medium flow channel may be referred to as saline flow channel, and so on. However, as for the "hole", "tube", and others are concerned, their structural characteristics are not strictly limited by the type of heat exchange medium, unless otherwise specifically indicated.

For example, if the heat exchange medium is physiological saline, the radio frequency ablation catheter in one of the embodiments includes an electrode 1 that is electrically connected to an ablation apparatus. One end of the electrode 1 has a saline connecting tube 101 that is connected to a saline tube 12 to introduce the physiological saline into the electrode 1. A saline hole 106 communicating with the interior of the saline connecting tube 101 is formed on the electrode 1. The electrode 1 is further provided with an infiltration cover 20 which is mounted around thereon, and a plurality of dispersing holes 200 are uniformly arranged on the infiltration cover 20, such that the physiological saline flowing out of the saline hole 106 can flow out through the dispersing holes 200. As shown in FIGs. 1 to 10c, a radio frequency ablation catheter according to an embodiment generally includes an insertion portion and a handle portion.

The insertion portion includes the electrode 1, a sheath 2 connected to the electrode 1, and components located inside the electrode 1 and the sheath 2.

The handle portion includes a saline joint 6 and an ablation apparatus connector 7. The handle portion is configured to connect the insertion portion to a physiological saline storage location connected with the saline joint 6 and to the ablation apparatus.

A lead wire 16 runs through the sheath 2 to connect the electrode 1 to the ablation apparatus connector 7, thereby connecting the electrode 1 with the ablation apparatus. The ablation apparatus is also connected to a neutral electrode pad. Before the ablation treatment starts, the neutral electrode pad is attached to a suitable site of the human body to form a circuit between the electrode 1, the ablation apparatus, the neutral electrode pad and the patient, thereby ablating the tissue in contact with the electrode 1. In use, the electrode 1 of the radio frequency ablation catheter is guided by bronchoscopic navigation to travel through the channel of the bronchoscope, and enter the lung parenchyma through a hole previously punctured in the bronchial wall near the lesion for radio frequency ablation.

When the infiltration cover 20 is provided, the dispersing device can be deemed as being formed separately from the electrode 1, and the dispersing hole 200 is the hole in the infiltration cover.

Of course, the dispersing device may be formed integrally with the electrode 1. For example, as shown in FIGs 2, 3 and 8, one end of the electrode 1 is connected and fixed to the sheath 2, and the other end is directly inserted in the tissue to be ablated. The end of the electrode 1 fixed to the sheath 2 is provided with a saline connecting tube 101 extending into the sheath 2, and the saline connecting tube 101 is connected to a saline joint 6 through a saline tube 12, to supply physiological saline to the electrode 1. As shown in FIG. 8, a plurality of saline holes 106 are formed on the electrode 1, and the physiological saline flowing out from the saline connecting tube 101 enters the electrode 1 and then enters the saline holes 106 via channels in the electrode 1 and flows out therefrom to achieve an infiltration effect on the surface of the electrode 1. A physiological saline layer is formed on the surface of the electrode 1. During ablation, the physiological saline is filled between the electrode 1 and the tissue, to avoid "scabbing" which may result in "vacuum enclosing" the electrode and thus causing suddenly increasing of the impedance.

The saline hole 106 shown in FIGs. 2, 3 and 8 can be deemed as the dispersing hole, and the portion of the electrode 1 with the saline hole 106 can be deemed as the dispersing device accordingly.

In case of a one-piece structure, to ensure the infiltration effect, a plurality dispersing holes are provided, which are preferably distributed in multiple groups such as 2 to 4 groups in the axial direction of the electrode. Each group has multiple dispersing holes, such as 2 to 8 and preferably 4 to 6, distributed along the circumferential direction of the electrode.

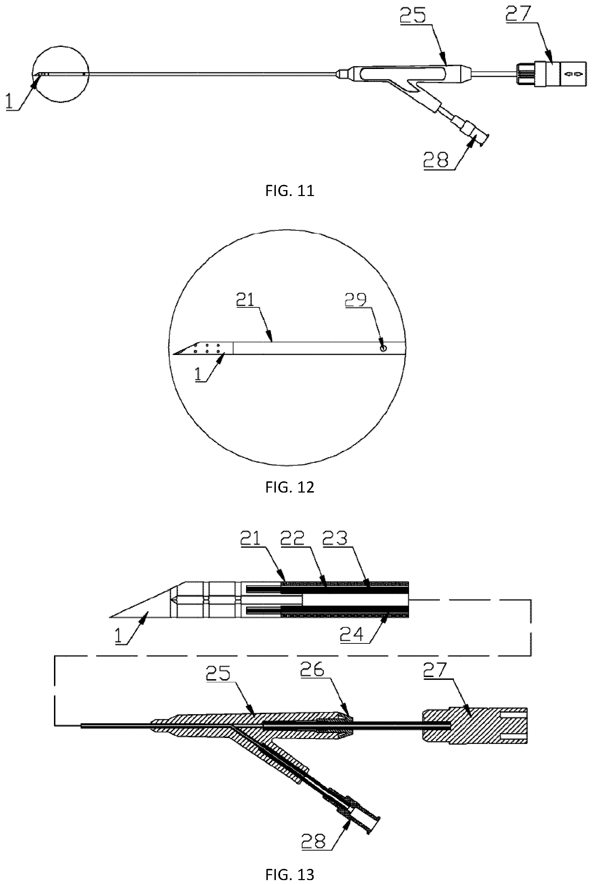

As shown in FIGs. 11 to 17, a radio frequency ablation catheter according to an embodiment generally includes an insertion portion and a handle portion.

The insertion portion includes an electrode 1, a metal tube 21 connected to the electrode 1, and components located inside the electrode 1 and the metal tube 21. Further, a temperature sensor is provided at a position near the electrode 1.

The shape of the distal end of the electrode 1 converges to form a sharp tip. The metal tube 21 is similar to the sheath 2 in structure and position, but the metal tube 21 has a certain strength, and may, together with the electrode 1, serve as a puncture needle. For example, it can be directly punctured into the lung through the outer wall of the chest cavity to implement the ablation.

In this embodiment, the dispersing device and the electrode are formed as one-piece structure 1. Heat exchange medium flow channels, such as a main flow channel 115, a branch flow channel 116, a branch flow channel 117, and a branch flow channel 118, are provided inside the electrode 1. The branch flow channels are communicated with the main flow channel 115 at different locations.

Each branch flow channel 116 extends to the outer surface of the electrode 1 to form a dispersing hole 109. The section of the electrode 1 having the heat exchange medium flow channels and the dispersing holes 109 can be deemed as the dispersing device which is integrated with the electrode 1.

The electrode 1 is provided a mounting hole 112 and a mounting hole 113, for accommodating the temperature sensor 22 and an electrode lead 24, respectively. A proximal end of the electrode 1 is provided with a connecting tube 111 which is communicated with the heat exchange medium flow channel inside the electrode 1. A heat exchange medium delivery pipe 23 is connected to the connecting tube 111 end to end.

The handle portion mainly includes a Y-shaped handle 25, a handle cover 26, and a Luer joint 28.

A circuit extends out of the Y-shaped handle 25 and then enters the connector 27. The connector 27 may adopt a common connector form to facilitate the pluggable connection to an external circuit. The heat exchange medium delivery pipe 23 may be connected to a heat exchange medium delivery apparatus via the Luer joint 28 to supply the heat exchange medium to the electrode 1.

According to the length of a path that the electrode 1 travels in the body, the proximal end of the electrode 1 may be connected with a member with a corresponding performance. For example, if the path in the body is longer and needs to be turned, a sheath 2 can be used. The sheath 2 has better flexibility and radial support ability, and can be adapted to a large extent of bending. In addition, the bending degree of the sheath and the angle of the electrode 1 can be adjusted by combining with a pull wire 10. For another example, if the path in the body is short, or if puncture is required, the metal tube 21 with a certain rigidity is used.

The electrode 1 and the sheath 2 or the metal tube 21 can be fixedly connected by traditional means, for example, welding, bonding, rivet connection or by using an intermediate connecting member. They can be connected axially end to end, or one is partially mounted around the other at the connection site. It is preferable that the outer wall is flat and smooth to avoid sharp edges and angles in case that one is mounted around the other one.

As shown in FIGs. 9 to 10d, to further optimize the infiltration effect to the electrode 1 during ablation, the dispersing device and the electrode are separately formed 1 in some embodiments. For example, an infiltration cover 20 is mounted outside the electrode 1 to serve as a dispersing device. There is a gap between the inner peripheral wall of infiltration cover 20 and the outer peripheral wall of the electrode 1.

A plurality of small dispersing holes 200 are evenly distributed on the infiltration cover 20. The physiological saline entering the channels in the electrode 1 from the saline connecting tube 101 flows to the gap between the electrode 1 and the infiltration cover 20 via the saline hole 106, and flows out from the dispersing holes 200, forming a thin water layer on the outer surface of the infiltration cover 20. In this way, the electrode surface is infiltrated by the physiological saline, thus further avoiding scabbing of the tissue ablated, reducing the circuit impedance, and maintaining balance of the impedance, to allow the ablation process to proceed continuously until the target volume of ablation is reached.

It is to be understood that the electrode 1 is often mechanically processed, and it is difficult to make the size of the saline hole 106 thereon very small, while the infiltration cover 20 may be processed in other ways, and the dispersing hole 200 thereon can be made to have a small diameter, so that the physiological saline flowing out of the dispersing hole 200 forms a water layer on the surface of the electrode. Regardless of the processing difficulty of the electrode 1, it is possible to form multiple saline holes 106 on the electrode 1, and all the saline holes 106 are evenly distributed on the surface of the electrode 1. As such, the infiltration cover 20 may not be provided.

The infiltration cover 20 can distribute the cooling medium to form a layer on the outer surface of the infiltration cover 20. To facilitate the processing and installation, the infiltration cover 20 and the electrode may be separately provided.

In one of the embodiments, the infiltration cover 20 is fixed to the electrode 1, rotatably mounted on the electrode 1 about the axis of the electrode 1, or slidably mounted on the electrode 1 along the axis of the electrode.

During assembly, the infiltration cover 20 is directly surrounding the electrode, or the infiltration cover 20 only covers a partial area of the electrode 1 and can be engaged in a predetermined area during installation. When the infiltration cover 20 is fixedly connected to the electrode 1, its position relative to the electrode 1 can be maintained by welding, or by a connecting member or a retaining structure.

The infiltration cover 20 may be mounted on the electrode 1, rotatable about the axis of the electrode. On the one hand, this requires only axial positioning; and on the other hand, by using the differentially distributed outflow holes in the circumferential direction, desirable outflow directions can be obtained by rotating the infiltration cover 20. It is even possible that outflow holes in some areas can be closed in this case.

The infiltration cover 20 may be slidably mounted on the electrode 1 along the axis of the electrode. As such, due to the differentially distributed outflow holes in the axial direction, the outflow of heat exchange medium in different areas can be adjusted by the movement of the infiltration cover 20. It is even possible that outflow holes in some areas can be closed in this case.

In order to control the movement of the infiltration cover 20 relative to the electrode 1, a driving member infiltration cover is further provided in one of the embodiments, which is connected to the infiltration cover 20 to drive the infiltration cover 20 to move relative to the electrode 1. For example, a pulling string that can move axially relative to the sheath 2 or a tube that can rotate relative to the sheath 2 may be adopted.

In one of the embodiments, one or more infiltration covers 20 are mounted on the electrode 1.

In case of one infiltration cover 20, it is possible that the infiltration cover 20 may cover only a partial area of the outer periphery of the electrode 1 in the circumferential direction. For example, the outer wall of the electrode 1 is provided with a recessed groove. The outflow hole is located at the bottom of the recessed groove. The infiltration cover 20 is mounted in the recessed groove and slightly spaced from the bottom of the recessed groove with a gap, to facilitate the distribution of the heat exchange medium.

In case of multiple infiltration covers 20, the multiple infiltration covers 20 may be arranged along the axial direction or circumferential direction of the electrode, and cover different areas of the electrode. When multiple infiltration covers 20 are provided, each infiltration cover 20 is mounted fixedly or movably independently of each other. For example, a portion of the infiltration covers 20 are fixed on the electrode 1, and a portion of the infiltration covers 20 are slidably or rotatably mounted on the electrode 1.

In one of the embodiments, multiple infiltration covers 20 are mounted on the electrode 1, and each infiltration cover 20 moves relative to the electrode 1 independently from each other. Alternatively, at least two infiltration covers are movable in association with each other.

The same infiltration cover 20 may have a one-piece structure, or a structure of multi-pieces that are fastened by, for example, snap-fit connection. Mounted independently means being fixed or movable independently, and movable in association with each other means that movement of one of them will drive or influence the other.

The infiltration cover 20 may also have a non-closed structure in the circumferential direction. In one of the embodiments, the infiltration cover 20 is in the shape of a sheet, which covers only a partial area of the outer periphery of the electrode 1 in the circumferential direction.

The sheet shape may be planar or a curved, and is fixed on the electrode 1 by engaging in or half-surrounding the electrode. As for the electrode 1, the infiltration cover 20 covers at least the area with the outflow hole. and of course, it may further extend circumferentially. In one of the embodiments, the infiltration cover 20 is of a cylindrical structure, which is circumferentially enclosed and mounted around the outer periphery of the electrode 1.

The infiltration cover 20 of cylindrical structure may be axially installed on the electrode 1 from the proximal side or distal side of the electrode during assembly. The circumferentially closed cylindrical structure allows the infiltration cover 20 to surround the electrode 1 along the whole circumference thereof, but is not required to surround all parts of the electrode in the axial direction. For example, in one of the embodiments, the infiltration cover 20 only surrounds the proximal portion of the electrode 1.

In one of the embodiments, the infiltration cover 20 has a cap-shaped structure with a distal end sealing and enclosing the distal tip of the electrode 1.

To facilitate the positioning and installation of the infiltration cover 20, in one of the embodiments, the infiltration cover 20 is fixed on the electrode 1. A positioning step is provided on the outer periphery of the electrode 1, and a distal end of the infiltration cover 20 is retained by abutting against the positioning step.

In order to make the outer wall of the distal portion of the radio frequency ablation catheter smooth and flat, in one of the embodiments, the outer wall of the infiltration cover 20 and the electrode 1 exposed from the outer wall of the infiltration cover 20 which are joined are flush with each other, avoiding the occurrence of local sudden changes and raised edges in shapes, thus reducing potential safety hazards.

At least a part of the infiltration cover 20 is a permeable area where the dispersing hole 200 is distributed, and the area of the electrode where the outflow hole is defined is corresponding to the permeable area, with a gap defined between it and the inner wall of the permeable area.

Due to the gap, the heat exchange medium is allowed to be distributed to improve the uniformity of the heat exchange medium protective layer. The gap may be formed by locally recessed from the outer wall of the electrode or locally protruding of the infiltration cover, or a combination of multiple ways.

In one of the embodiments, the dispersing hole 200 and the outflow hole are arranged in an offset manner.

The arrangement in an offset manner may at least avoid one-to-one alignment, whereby the heat exchange medium is prevented from preferentially flowing out from the dispersing hole right opposite to the outflow hole, impacting the distribution performance of the infiltration cover 20. Similarly, it is preferable to have a larger number of dispersing holes 200 with a smaller diameter than the diameter of the outflow hole.

Referring to FIG. 10b, in one of the embodiments, the outer wall of the electrode 1 is provided with a recessed area 107, the outflow hole is arranged in the recessed area 107. The permeable area of the infiltration cover 20 is located at an outer periphery of the recessed area 107, with a gap defined between the inner wall of the infiltration cover 20 and the surface of the recessed area 107.

The arrangement of the recessed area 107 allows for further even distribution of the heat exchange medium output from each outflow hole before the heat exchange medium permeates out through the infiltration cover 20. The size of the gap impacts the pressure or flow rate of the heat exchange medium on different areas of the infiltration cover 20. To obtain a uniform output of the heat exchange medium on the outer periphery of the infiltration cover 20, for example, to form a uniform physiological saline layer, the gap changes accordingly as the distance from the adjacent outflow holes varies.

Referring to FIG. 10d, in one of the embodiments, the outflow hole is flared, and the flared area serves as the recessed area. The gap between the inner wall of the infiltration cover and the surface of the recessed area decreases as the distance from the outflow hole increases.

The flared shape, i.e., forming a trumpet-shaped opening, acts to uniformly output the heat exchange medium. For example, when the heat exchange medium has a high flow rate, it is easy to directly eject from the nearest dispersing hole, making it impossible for the dispersing hole far away from the outflow hole and the dispersing hole near the outflow hole to output the heat exchange medium uniformly, which affects the uniformity of the heat exchange medium protective layer.

In FIG. 10d, the opening of the outflow hole, that is, the saline hole 106, in the outer wall of the electrode 1 is in a flared form, and the flared area serves as the recessed area 107. The gap between the inner wall of the infiltration cover 20 and the surface of the recessed area decreases as the distance between the inner wall of the infiltration cover 20 and the outflow hole increases. The flared configuration can increase the distance between the outflow hole and the dispersing hole opposite to it, alleviate ejection of the heat exchange medium, and allow the dispersing holes with different distances from the outflow hole to output the heat exchange medium uniformly. For another example, when the heat exchange medium has a relatively slow flow rate, the dispersing hole far away from the outflow hole has a lower flow rate than the dispersing hole near the outflow hole due to insufficient supply. Therefore, in one of the embodiments, the gap between the inner wall of the infiltration cover 20 and the surface of the recessed area increases as the distance from the outflow hole increases.

After the heat exchange medium to be output from the outflow hole is distributed in the recessed area, sufficient heat exchange medium supply can be ensured due to the large gap at a position away from the outflow hole, so that the flow rate in each dispersing hole is relatively balanced on the whole to form a uniform heat exchange media protective layer.

If multiple outflow holes are present around a certain area between the inner wall of the infiltration cover 20 and the surface of the recessed area, the distance between the area and the outflow hole may be calculated by taking the multiple adjacent outflow holes into consideration, for example, it may be calculated based on the average distance.

In one of the embodiments, one recessed area is provided, or more recessed areas isolated from each other are provided. One outflow hole is provided in one recessed area. In one recessed area, the gap between the inner wall of the infiltration cover 20 and the surface of the recessed area increases as the distance from the outflow hole in the recessed area increases.

Referring to FIG. 10b, for example, as for the recessed area 107 corresponding to the saline hole 106, the depth of the recessed area 107 varies as the distance between it and the corresponding saline hole 106 varies, and the longer the distance from the saline hole 106 is, the deeper the recessed area 107 would be.

To facilitate the calculation and processing, in one of the embodiments, the recessed area is a distribution groove extending along the axis of the electrode. Several groups of outflow holes (for example, saline hole 106 as shown) are arranged in the circumferential direction of the electrode, and each group corresponds to a respective distribution groove.

That is, the outflow holes of a same group are defined at the bottom of a same distribution groove, and the depth of the distribution groove can be deemed as the size of the gap between the inner wall of the infiltration cover 20 and the surface of the recessed area.

With reference to the drawings, in one of the embodiments, 2 to 10, for example, 4, 6, or 8, distribution grooves are evenly arranged in the circumferential direction. The infiltration cover 20 is cylindrically surrounding the periphery of all distribution grooves.

The number of outflow holes in a same group may be one, or more in which the outflow holes arranged in sequence, for example, one saline hole 106 is provided as shown in the figure.

In one of the embodiments, one outflow hole is defined at the bottom of one distribution groove, and the depth of the distribution groove increases as the distance from the outflow hole increases.

In one of the embodiments, multiple groups of dispersing holes 200 are provided along the circumferential direction on the infiltration cover 20, and each group of dispersing holes 200 correspond, in position, to one of the distribution grooves.

When processed mechanically, the number of the dispersing holes 200 corresponding to a same distribution groove may be one, or multiple arranged in sequence, such as 4 to 10 as shown in the figure. If a woven or a porous material is used, the number and distribution of dispersing holes 200 are relatively complicated. Therefore, the corresponding relationship of them and the distribution groove is mainly considered in the principle of grouping.

The wall between adjacent distribution grooves forms a rib supporting the inner wall of the infiltration cover 20, and the top of the rib abuts against and has a shape fitting with a corresponding area on the inner wall of the infiltration cover 20.

For example, if the infiltration cover 20 is a cylinder, then the rib has a curved surface with a curvature corresponding to that of the infiltration cover 20, thereby providing a better support and reducing undesirable local deformation of the infiltration cover 20.

Dispersing holes 200 are provided on the infiltration cover 20. Depending on the processing methods of the infiltration cover 20, the dispersing holes 200 have corresponding distribution characteristics and shapes.

In one of the embodiments, the infiltration cover 20 is formed by a porous material, and the inherent pores of the porous material serve as the dispersing holes 200.

The porous material can be engaged in a part of the electrode 1, or formed with a cylindrical or cap shape to be mounted on the electrode 1, to cover at least the saline hole 106 in an expected area. As for the porous material, those known in the prior art, for example, foam metal, can be used.

In one of the embodiments, the infiltration cover 20 adopts a woven structure, where the inherent pores of the woven structure serve as the dispersing holes 200.

When woven, for example, warps and wefts of fiber material are interlaced, with pores formed between the warps and wefts, which serve as the dispersing holes 200. As for fiber material, for example, Nitinol or the like can be used.

In one of the embodiments, the infiltration cover 20 is a metal housing, and the dispersing holes 200 are formed on the wall of the metal housing.

The diameter and density distribution of the dispersing hole can be set as required by the flow rate of the heat exchange medium, to ensure as far as possible that a uniform protective layer is formed on the other periphery of the electrode. For example, the diameters of all dispersing holes are the same, or the corresponding arrangements are balanced according to the flow rate of the heat exchange medium.

That is, the diameters of the dispersing holes in different areas may be different to meet the needs of balanced flow rate. Likewise, all dispersing holes have the same distribution density in different areas of the dispersing device, or the corresponding settings are balanced according to the flow rate of the heat exchange medium.

When the corresponding settings are balanced according to the flow rate of the heat exchange medium. For example, the diameter of the dispersing hole increases as the distance between it and the outflow hole increases.

Similarly, for example, the distribution density of the dispersing hole increases as the distance from the outflow hole increases.

The dispersing holes can be arranged as desired during processing. For example, in one of the embodiments, the dispersing holes 200 are distributed in multiple groups, for example, 2 to 6 groups, in the circumferential direction of the infiltration cover 20.

The number of dispersing holes in each group is 2 or more, for example 4 to 10. On the one hand, the dispersing holes may be grouped according to the trend of arrangement, or according to the corresponding relationship with the heat exchange medium flow channel.

The dispersing holes 200 in a same group are arranged in sequence according to the respective extension path, which is straight, zig-zag or curved. When the extension path is straight, the dispersing holes 200 in the same group can be deemed as being arranged in sequence along the axial direction. When the extension path is curved, the overall orientation can be extension along the axis, while a specific path can be an arc, or an S-shaped curve, etc.

In other embodiments, all the dispersing holes 200 may be distributed spirally around the axis of the electrode, or the dispersing holes 200 are evenly distributed in an array.

In order to achieve balanced water output from the infiltration cover 20 and to form a uniform water layer, in one of the embodiments, the diameters, also understood as areas of the cross sections, of the dispersing holes 200 in a same group, increase as the distances from the outflow hole increase.

If there are multiple outflow holes around the dispersing holes 200 in the same group, only the closest outflow hole is considered or it can be calculated according to the average distance. In one of the embodiments, each group of dispersing holes 200 corresponds to one outflow hole. In this case, the change in diameter of the dispersing holes 200 of the same group can only consider the corresponding outflow hole in the circumferential direction.

As shown in FIG. 10, multiple dispersing holes such as dispersing hole 201 and dispersing hole 202 correspond to the saline hole 106 in the circumferential direction. The dispersing hole 202 is farther away from the saline hole 106 than the dispersing hole 201. The diameter of the dispersing hole 202 is correspondingly larger than that of the dispersing hole 201.

In one of the embodiments, the infiltration cover 20 has a developing mark.

At least a part of the infiltration cover 20 itself is formed of a developing material or a developing mark is provided on the infiltration cover 20. The developing mark can work with an imaging device to indicate the position of the electrode 1 during operation.

The shape of the developing mark is not strictly limited, for example, a ring shape extending in the circumferential direction, a C shape or a strip shape extending in a predetermined direction can be adopted.

In one of the embodiments, multiple developing marks are arranged on the infiltration cover 20 sequentially along the axial direction. The multiple developing marks arranged in sequence along the axial direction further facilitate the identifying of the spatial posture. For example, the relative inclination to the viewing angle can be determined according to the length of the lined developing marks, or the spatial position of the electrode can be determined in combination with the curvature of the lined developing marks.

In one of the embodiments, the radio frequency ablation catheter further includes an electromagnetic navigation member that can indicate the position of the electrode.

The electromagnetic navigation member may be those used in the prior art. A mounting hole can be formed in the electrode to accommodate the electromagnetic navigation member, or the electromagnetic navigation member can be attached and fixed to an outside of the electrode. During the intervention and ablation, the position of the electromagnetic navigation member in the body can be detected by an imaging device. In this way, the position or posture of the electrode is determined, to guide and monitor the ablation operation.

The electrode travels in the body. During the ablation operation, the orientation or spatial posture of the electrode is needed to be adjusted sometimes. Generally, a pull wire is used for pulling at the distal end.

A radio frequency ablation catheter according to an implementation includes an electrode 1, and a pull wire 10 extending towards the proximal end is connected to the electrode 1 to drive the electrode 1 to deflect.

The use of the pull wire 10 facilitates the adjustment of the electrode 1, and on this basis, related features (or implementation) of the aforementioned infiltration cover 20 may be combined. For example, a heat exchange medium flow channel is provided inside the electrode 1, an outflow hole communicating with the heat exchange medium flow channel is defined on an outer wall of the electrode 1, and an infiltration cover 20 is mounted on the electrode 1 at an outer periphery at the outflow hole. A heat exchange medium output from the outflow hole flows out via the infiltration cover 20.

In order to introduce a heat exchange medium into the electrode 1, a proximal end of the electrode 1 is provided with a connecting tube communicating with the heat exchange medium flow channel. The inside of the connecting pipe may be deemed as a part of the heat exchange medium flow channel. The connecting pipe may be integrally formed with the electrode, to connecting to an external pipeline and extend to the proximal end.

In one of the embodiments, the proximal end of the electrode is connected to a sheath 2 made of a flexible material, and the pull wire 10 extends inside the sheath 2 towards the proximal end to reach the outside of the sheath 2. The proximal end of the electrode 1 is provided with a connecting tube that communicates with the heat exchange medium flow channel. The connecting tube extends to the inside of sheath 2.

In order to install a pull wire 10 to facilitate the manipulation of the electrode 1, in one of the embodiments, and one end of the electrode 1 is connected with a sheath 2 made of a flexible material. A saline connecting tube 101 extends to the inside of the sheath 2. The pull wire 10 is fixedly provided on the electrode 1. The pull wire 10 extends from the inside of the sheath 2 to the outside of the sheath 2, so that when the pull wire 10 is pulled, the sheath 2 bends and deforms, thereby driving the electrode 1 to deflect.

The pull wire 10 extending from the inside of the sheath 2 to the outside of the sheath 2 can be understood as extending from the inside of the sheath 2 towards the proximal end until it extends out of the proximal end of the sheath 2. Of course, it can also extend through the wall of the sheath 2 radially at a position near the proximal portion of the sheath 2, to extend to the outside of the sheath 2.

In one of the embodiments, the radio frequency ablation catheter further includes a first stretch bending component 4 and a second stretch bending component 5 that can move relatively close to or away from each other. The sheath 2 is fixed to the first stretch bending component 4, and the pull wire 10 is fixed to the second stretch bending component 5.

That is, the handle portion further includes the first stretch bending component 4, and the second stretch bending component 5 slidably fitting with the first stretch bending component 4. The electrode 1 is controlled to deflect towards a target position by the relative movement between the first stretch bending component 4 and the second stretch bending component 5.

The first stretch bending component 4 and the second stretch bending component 5 each maybe a single piece or a combination of multiple pieces, or they may even be different portions of a same component.

In one of the embodiments, the first stretch bending component 4 and the second stretch bending component 5 are arranged slidably by means of that one is slidably aranged in the other, or arranged slidably side by side. In order to improve the stability of the relative movement, necessary guiding and retaining structures may be provided.

To fix the proximal end of the pull wire 10, a mounting hole is provided on the electrode 1 in one of the embodiments, and the distal end of the pull wire 10 is inserted in and fixed to the mounting hole. By means of the mounting hole, the end of the pull wire can be prevented from being exposed, and the connection strength can be improved. When being fixed, welding, bonding or a combination thereof can be used, or an anchor is fixed at one end of the pull wire which is in interference fit with the installation hole. Alternatively, the end of the pull wire is fixed to the electrode 1 by an intermediate connecting member.

The opening of the mounting hole is at the proximal end of the electrode. To improve the connection strength, the mounting hole may extend over a distance along the axial direction of the electrode toward the distal end, and the pull wire 10 can be further inserted to the bottom of the mounting hole towards the distal end.

In the figures, one end of the pull wire 10 is fixed in a second mounting hole 103 on the electrode 1, and the other end is extended along the sheath 2 and fixed to the second stretch bending component 5. When the first stretch bending component 4 and the second stretch bending component 5 are moved relatively one another, the pull wire 10 is pulled, so that the sheath 2 is pulled and bent, causing the electrode 1 at the end of the sheath 2 to deflect. In some embodiments, a spring hose 11 is provided around the pull wire 10. When the pull wire 10 is pulled, the spring hose 11 can be bent and deformed with the sheath 2, and can restore after the pulling of the pull wire 10 is released.

When the pull wire 10 is tensioned by a force, the members adjacent thereto may be exerted with a greater pressure or even ruptured. The spring hose 11 can further provide buffer and protection.

As shown in FIGs. 4 to 7, the sheath 2 extends from one end of the electrode 1 towards the handle portion, and is fixed to the first stretch bending component 4. The sheath 2 is made of a material with certain flexibility, so that it is able to cause an elastic deformation which is recoverable, thereby controlling the deflection of the electrode 1.

In some implementations, to strengthen the connection between the sheath 2 and the first stretch bending component 4, a protective tube 3 is mounted around and fixed on a connecting joint 402 of the first stretch bending component 4, and a part of the protective tube 3 extend to the outside of the sheath 2.

In one of the embodiments, the first stretch bending component 4 and the second stretch bending component 5 are both tubular, and the second stretch bending component 5 is slidably fitted inside the first stretch bending component 4.

A sliding chamber 401 is formed inside the first stretch bending component 4, and the sliding chamber 401 is provided with an opening at an end away from the end fixed to the sheath 2. The second stretch bending component 5 is slidably engaged in the sliding chamber 401 via the opening. The part of the second stretch bending component 5 that is slidably engaged in the sliding chamber 401 constitutes a sliding tube body 501. A wire-running cavity 500 is formed in the sliding tube body 501, and the pull wire 10 and a lead wire 16 extending from the sheath 2 run through the wire running cavity 500. An end cover 14 is provided on the second stretch bending component 5 at an end away from the end fixed to the sheath 2, and the wires running through the wire running cavity 500 passes through the end cover 14 and are respectively connected to the saline joint 6 and the ablation apparatus connector 7.

A bolt hole 503 is defined on the outer wall of the second stretch bending component 5, and a pull-wire fixing bolt 13 is screwed in the bolt hole 503. The pull wire 10 is fixed to the second stretch bending component 5 by the pull wire fixing bolt 13. In this way, when the sliding tube body 501 slides in the sliding chamber 401, the end of the pull wire 10 away from the electrode 1 moves with the sliding tube body 501, thereby pulling the sheath 2 to bend, and driving the electrode 1 installed at the end of the sheath 2 to deflect.

In some implementations, at least part of the second stretch bending component 5 is inserted in the first stretch bending component 4, and a guiding means for limiting the relative movement direction is further provided between the first stretch bending component 4 and the second stretch bending component 5.

The guiding means is provided between the first stretch bending component 4 and the second stretch bending component 5 to limit the sliding tube body 501 to cause only axial relative displacement without relative rotation in the sliding chamber 401.

In some implementations, the guiding means includes a sliding groove 502 arranged on either one of the first stretch bending component 4 and the second stretch bending component 5, and a limit screw 403 arranged on the other one.

Specifically, the first stretch bending component 4 is provided with the limit screw 403 extending to the sliding chamber 401. The sliding groove 502 is defined on the outer wall of the sliding tube body 501. When the second stretch bending component 5 is slidably engaged in the sliding chamber 401 of the first stretch bending component 4, the limit screw 403 is at least partially located in the sliding groove 502. In this way, the relative movement direction of the first stretch bending component 4 and the second stretch bending component 5 is limited by means of the sliding limit effect of the sliding groove 502 on the limit screw 403. The length of the sliding groove 502 also limits the maximum bending degree of the sheath 2.

Those skilled in the art can understand that there are many ways to limit the relative movement direction of the first stretch bending component 4 and the second stretch bending component 5, and the present invention is not limited to the above-mentioned manner of cooperation of the limit screw 403 and the sliding groove 502.

In some implementations, the part of the second stretch bending component 5 inserted in the first stretch bending component 4 is provided with an O-ring 15, for increasing the friction between the first stretch bending component 4 and the second stretch bending component 5.

For example, the outer peripheral wall of the sliding tube body 501 is provided with a groove 504 extending along a perimeter of the sliding tube body 501, and the O-ring 15 is mounted in the groove 504. In this way, during the relative movement of the first stretch bending component 4 and the second stretch bending component 5, the O-ring 15 can appropriately increase the contact friction between the sliding tube body 501 and the sliding chamber 401, to increase the hand feeling when the two stretch bending components move relative to each other. Therefore, since a force is required to pull the pull wire 10 in controlling the deflection angle of the electrode 1, it is easier to control the distance that the pull wire 10 is pulled, so that the bending degree of the sheath 2 can be controlled more accurately, and the deflection angle of the electrode 1 can be controlled more accurately accordingly.

To further improve the convenience in driving the relative movement between the first stretch bending component 4 and the second stretch bending component 5, the first stretch bending component 4 is preferably provided with two rings 400, and the second stretch bending component 5 is further provided with two rings 400. The fingers of an operator can pass through the rings 400 to form a fixation with either one of the stretch bending components, so as to drive the two stretch bending components to move relatively. It is also convenient for single hand operation.

To further improve the accuracy of driving the relative movement between the first stretch bending component 4 and the second stretch bending component 5, in one of the embodiments, the second stretch bending component 5 has a scale mark provided thereon, to indicate the position relative to the first stretch bending component 4.

In one of the embodiments, the electrode 1 is further provided with a thermistor 9 for detecting the real-time temperature of the electrode 1. The thermistor 9 is electrically connected to the ablation apparatus through a thermistor wire 90.

The thermistor 9 is fixed on the electrode 1 to obtain the real-time temperature data of the tissue being ablated, and transmit the temperature data to the ablation apparatus by the thermistor wire 90 connecting the thermistor 9 to the connector 7 of the ablation apparatus.

In one of the embodiments, a temperature control sleeve 91 is provided in the sheath 2, mounted around the thermistor wire 90.

That is, to prevent the thermistor wire 90 from being disturbed, the temperature control sleeve 91 is provided outside the thermistor wire 90. The temperature control sleeve 91 is mounted outside the thermistor wire 90.

In one of the embodiments, an electrode ring 8 is further provided outside the sheath 2, and a temperature sensor 19 is provided in the sheath 2. The electrode ring 8 is connected to the electrode ring 8 and capable of detecting the temperature of the electrode ring 8. The temperature sensor 19 is electrically connected to the ablation apparatus.

The electrode ring 8 is arranged on the outer wall of the sheath 2 at a position about 2 cm away from the electrode 1. A via hole is defined on the outer wall of the sheath 2 at the position where the electrode ring 8 is provided, and the via hole communicates with the inner cavity of the sheath 2. A bifurcated riveting tube 18 is provided in the sheath 2. A part of the bifurcated riveting tube 18 extends through the via hole and is welded and fixed to the electrode ring 8, and another part of the bifurcated riveting tube 18 located in the inner cavity of the sheath 2 is connected to the temperature sensor 19. The temperature sensor 19 is electrically connected to the connector 7 of the ablation apparatus. As such, the temperature sensor 19 detects the temperature of the electrode ring 8 through the bifurcated riveting tube 18, and transmits the temperature to the ablation apparatus. During an ablation process, when the range of ablation reaches the electrode ring 8 (generally the diameter of ablation range is 2 cm), the temperature detected by the temperature sensor 19 reaches a preset value. At this time, it can be determined that the range of ablation has reached a preset value, and the ablation apparatus is stopped to output energy to the electrode 1, so as to stop the ablation process. Those skilled in the art can understand that according to actual needs, the radius of ablation range can be controlled by moving the position of the electrode ring 8 along the sheath 2 and correspondingly changing the preset temperature, thereby effectively controlling the size of the ablated area.

The electrode ring 8 may be a, in the circumferential direction, a closed whole ring, or have a non-enclosed structure such as a C-shape structure or a structure with a smaller wrap angle. To facilitate the installation of the electrode ring 8, a patch form may be used. The shape of patch is not strictly limited, and the patch may be fixed on the outer wall of the electrode by for example welding. The electrode ring 8 is a heat-conducting element. The temperature sensor 19 may be a thermistor in a circuit, to detect the temperature by the changes of electrical signals.

In the prior art, it is difficult to directly determine the ablation effect, and are mostly evaluated by surgery. There is no determination or evaluation method available during the operation. In this application, in a radio frequency ablation catheter according to an implementation, multiple temperature detection devices are provided in sequence along the axial direction in an area adjacent to the distal end of the radio frequency ablation catheter.

The multiple temperature detection devices can acquire the surrounding temperatures of the electrode 1. During the ablation process, the surrounding temperature has a gradient change centered on the electrode. Therefore, the temperature in the center of the ablated region can be acquired (monitored in real time) and the ablation status at the edge of the region can be obtained.

The multiple temperature detection devices may be implemented alone or in combined with one or more of the pull wire 10 and the infiltration cover 20 and related features (or implementations). For example, a pull wire 10 extending distally is connected to the electrode 1 to drive the electrode 1 to deflect. By monitoring the temperature, the electrode 1 can be driven to deflect by the pull wire 10 when necessary.

For another example, a heat exchange medium flow channel is provided inside the electrode 1, an outflow hole communicating with the heat exchange medium flow channel is defined on an outer wall of the electrode 1, and an infiltration cover 20 is mounted on the electrode 1 at an outer periphery at the outflow hole. A heat exchange medium output from the outflow hole flows out via the infiltration cover 20. By monitoring the temperature, the output of the heat exchange medium can be adjusted in time, to form a heat exchange medium protective layer after passing through the infiltration cover 20.

For example, 2, 3, or more temperature detection devices may be provided, and the acquired temperature signals can be visually displayed in real time to guide the ablation operation. This makes it possible to effectively control the range of ablation, and directly determine or estimate the ablation effect during the operation.

In one of the embodiments, the distal end portion of the electrode 1 is equipped with a temperature probe, for acquiring the temperature of the distal end portion of the electrode 1. The distal end portion of the electrode 1 can be delivered to a position corresponding to the center of the lesion during ablation. For convenience of intuitive comparison, the relative positional relationship and relative temperature relationship of other temperature detection devices are made with reference to the distal end portion of the electrode 1.

The temperature probe may be connected to the ablation apparatus wirelessly or wiredly. For example, a mounting engaging groove can be provided at the distal end portion of the electrode 1, and the temperature probe is engaged in the mounting engaging groove.

When connected wiredly, a lead wire channel (bypassing the heat exchange medium channel) can be opened inside the electrode 1 for allowing the lead wire to run to the ablation apparatus.

To facilitate the installation of the temperature detection device and the temperature detection, the direct temperature-sensing element (for example, electrode ring 8) of the temperature detection device needs to be arranged outside of the electrode 1 or exposed from the electrode 1. Unless otherwise specifically stated hereinafter, the description concerning the axial relative position of the temperature detection devices therebetween and of the temperature detection device and the electrode should be understood as that regarding the direct temperature-sensing elements, which may be such as a ring-shaped, sheet-shaped, or post-shaped heat-conducting element, by which the temperature is transferred to the temperature sensor (which may be a thermistor and the like in the circuit). It is also possible that the temperature sensor is directly used as the temperature-sensing element and exposed from the electrode 1 (i.e., outside the radio frequency ablation catheter). The specific shape is not strictly limited. Of course, some preferred or improved implementations are provided below.

In one of the embodiments, the proximal end of the electrode 1 is connected end to end to the sheath 2 or the metal tube 21, and the temperature detection device is installed outside the electrode 1, outside the sheath 2, or outside the metal tube 21.

The sheet-shaped or rode-shaped temperature detection device can be engaged in the outer wall of the electrode 1. The ring-shaped temperature detection device can be fixed around the outside of the electrode 1, the sheath 2 or the metal tube 21. The ring-shaped temperature detection device may be circumferentially closed, or non-enclosed (for example, C-shaped).

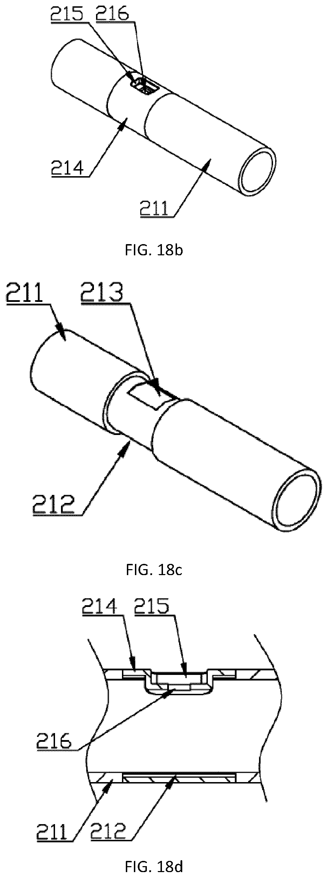

The temperature detection device may be installed movably or fixedly. Referring to FIG. 18c, when installed fixedly, the temperature detection device in one of the embodiments includes a temperature sensor (not shown). The outer wall of the radio frequency ablation catheter 211 is provided with an engaging groove 212, the temperature sensor is fixed in a respective engaging groove 212 at a corresponding position, and the bottom of the engaging groove 212 is provided with a first through hole 213 for running the circuit wire.

Any one or more temperature detection devices may be fixedly installed. The above manner of installation is for one of them. When multiple temperature detection devices are fixedly installed, the specific manners of installation and fixation may be the same or different.

Depending on the positions of the temperature detection devices, the shown radio frequency ablation catheter 211 may be a part of the electrode 1, the sheath 2 or the metal tube 21 in the foregoing embodiments. The depth of the engaging groove 212 is corresponding to the dimension of the temperature sensor in the radial direction. The shape of the engaging groove 212 is corresponding to that of the temperature sensor. That is, the temperature sensor can be fully filled in the engaging groove 212 to avoid undesired gaps.

In one of the embodiments, the engaging groove 212 is annular, and the temperature sensor is circumferentially fixed in a respective engaging groove 212 at a corresponding position. The fixing means between the temperature sensor and the engaging groove 212 is at least one selected from bonding, welding, riveting, and interference fit. For example, the engaging groove 212 as shown extends along one perimeter of the radio frequency ablation catheter 211, and the corresponding temperature sensor is ring shaped. They are fixed in one or more ways, without limitation. Of course, the engaging groove 212 may also have a circumferentially non-closed structure, such as C shaped.

The temperature sensor may be in the form of a patch, and is directly used as a temperature sensing element and exposed at the outer wall of the radio frequency ablation catheter 211, such that the acquisition of temperature signals is more timely and more accurate.

When the area or volume of the temperature sensor is small, for convenience of installation, the temperature detection device in one of the embodiments includes a temperature sensor (not shown) and a heat conducting ring 214, as shown in FIGs 18b to 18d. The temperature sensor is connected to the ablation apparatus, and the heat conducting ring 214 is arranged on the outer wall of the radio frequency ablation catheter 211. The temperature sensor is thermally coupled to the heat conducting ring 214.

The thermal coupling can be direct contact or indirect contact, as long as temperature can be transferred therebetween. The heat conducting ring 214 is not strictly limited to a complete ring shape, and may be a segment extending in the circumferential direction. The complete ring shape is circumferentially closed, such as a circular ring, and the segment extending in the circumferential direction is circumferentially non-closed, such as a C-shape.

In one of the embodiments, the temperature sensor is fixed on the outer wall of the heat conducting ring, where the fixation means is at least one selected from bonding, welding, riveting, and interference fit.

In one of the embodiments, the temperature sensor is a thermistor, which is electrically connected to the ablation apparatus by a thermistor wire. A temperature control sleeve is mounted around the thermistor wire. The temperature control sleeve can prevent the thermistor wire from being interfered. The temperature control sleeve is generally closely attached to the thermistor wire and is inside the radio frequency ablation catheter.

To facilitate the transmission, the circuit wire is omitted. In one of the embodiments, the temperature sensor is connected to the ablation apparatus by a wireless communication device.

In one of the embodiments, the outer wall of the radio frequency ablation catheter 211 is provided with an engaging groove 212, the temperature detection device is fixed in a respective engaging groove 212 at a corresponding position, and the bottom of the engaging groove 212 is provided with a through hole for running the circuit wire.

The bottom of the engaging groove 212 is provided with a first through hole 213. In the temperature detection device, a part of the heat conducting ring 214 is engaged into the first through hole 213, and the engaged part of the heat conducting ring 214 is provided with a second through hole 216. The circuit wire of the temperature sensor extends into the radio frequency ablation catheter 211 via the second through hole 216 and extends towards the proximal end.

To facilitate the positioning of the heat conducting ring 214 and prevent the rotational or axial misalignment, in one of the embodiments, the engaging groove 212 is annular, and the temperature detection device is fixed around in the engaging groove 212 at a corresponding position. The fixing means of the temperature detection device to the engaging groove 212 is at least one selected from bonding, welding, riveting, and interference fit.

In one of the embodiments, the heat conducting ring 214 has a ring structure, and the heat conducting ring 214 is arranged in the engaging groove 212. The heat conducting ring 214 has a complete ring structure, and the temperature sensor is further fixed on the inner wall or outer wall of the heat conducting ring 214.

In one of the embodiments, the temperature detection device has a ring structure, and the heat conducting ring 214 and the temperature sensor are circumferentially complementary in shape to together form the ring structure. In this embodiment, the heat conducting ring 214 and the temperature sensor are joined to a complete ring in the circumferential direction. For example, they both are semicircular rings. For example, the heat conducting ring 214 is C-shaped, and the temperature sensor is of C shape just fills the gap, that is, they are complementary.

In one of the embodiments, a recessed groove 215 is provided on the heat conducting ring 214, and the temperature sensor is fixed in the recessed groove 215. The fixing means of the temperature sensor to the recessed groove 215 is at least one selected from bonding, welding, riveting, and interference fit.

A part of the heat-conducting ring 214 is engaged into the first through hole 213, and this part correspondingly forms the recessed groove 215 outside the heat-conducting ring 214. The temperature sensor is fully filled in the recessed groove 215 to avoid undesired gaps.

To make the outer surfaces of the electrode 1 and the sheath 2 smooth and free of sharp edges, in one of the embodiments, the outer surface of the temperature detection device is flush with surrounding area.

In one of the embodiments, at least one of the temperature detection devices is located in the middle of the electrode 1 in the axial direction.

During ablation, a temperature field is formed centered on the electrode, and at least one of the temperature detection devices is at the center of the temperature field. It can not only detect the temperature change in the area, but also mark and determine the distribution of the temperature field by cooperation with other temperature detection devices according to the difference therebetween.

In one of the embodiments, at least one of the temperature detection devices is fixed around the outer periphery of the radio frequency ablation catheter.

The specific area where the temperature detection device is fixed around may be the outer wall of the electrode 1, the sheath 2 or the metal tube 21.

In one of the embodiments, the temperature detection devices include a first temperature detection device, a second temperature detection device, and a third temperature detection device arranged at intervals from the distal end towards the proximal end, wherein
the first temperature detection device is located at the distal end portion of the electrode 1;
the second temperature detection device is fixed around an outside of the electrode 1, the sheath 2 or the metal tube 21, and is located at the proximal end of the first temperature detection device; and
the third temperature detection device is fixed around an outside of the electrode 1, the sheath 2 or the metal tube 21, and is located at the proximal end of the second temperature detection device.

For example, the first temperature detection device is sheet-shaped or post-shaped, and the second temperature detection device and the third temperature detection device are ring shaped. In case of a sheet-shaped structure, the temperature detection device can be fixed on the outer wall of the electrode 1, the sheath 2 or the metal tube 21 in the form of a patch.

Depending on the axial length of the electrode 1, when the electrode 1 is short, the second temperature detection device and the third temperature detection device are both arranged outside the sheath 2 or the metal tube 21. When the electrode 1 is slightly longer, the second temperature detection device is arranged outside the electrode 1, and the third temperature detection device is arranged outside the sheath 2 or metal tube 21. When the electrode 1 is further elongated, the second temperature detection device and the third temperature detection device are both arranged outside the electrode 1.

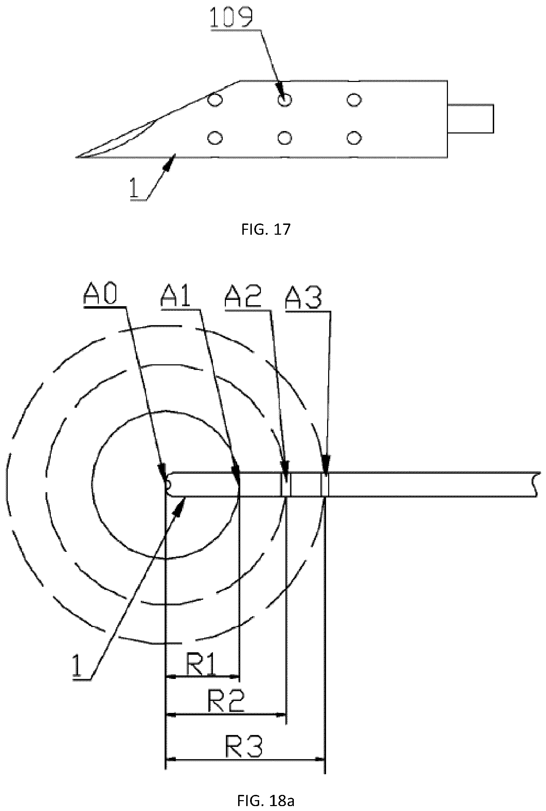

Referring to FIG. 18a, the detection position corresponding to the temperature probe at the distal end portion of the electrode 1 is A0, the detection position corresponding to the first temperature detection device is A1, the detection position corresponding to the second temperature detection device is A2, and the detection position corresponding to the third temperature detection device A3.

A0 may be deemed as the heat center, that is, the site with the highest temperature. The temperature will gradually decrease as the distance from A0 increases. For example, the temperature in an area with a radius of R1 (for example, 1 cm) may reach 100°C; the temperature at the edge of an area with a radius of R2 (for example, 1.5 cm) may generally reach 43°C to 60°C, which can still meet the requirements of ablation treatment, but the temperature at the edge of an area with a radius of R3 (for example, 2 cm) is further reduced and cannot meet the requirements of ablation treatment, which is only for monitoring for reference.

To match the temperature field with the volume of the lesion site, when multiple temperature detection devices are used, the multiple temperature detection devices can be fixed in advance. That is, radio frequency ablation catheters of different specifications can be chosen according to the different distances. In practical use, a radio frequency ablation catheter of appropriate specification is selected according to the volume of lesion site known previously.

The shape and size of the lesion sites to be ablated are different. For example, to improve the compatibility, the axial position of at least one of the temperature detection devices is adjustable in one of the embodiments.

The detection position relative to the distal end of the electrode can be changed by changing the position of the temperature detection device. For example, the axial position of the second temperature detection device in the above embodiment is adjustable, or the axial positions of both the second temperature detection device and the third temperature detection device are both adjustable.

Referring to FIG. 18a, when the axial position of the second temperature detection device changes, the distance between A1 and A2 changes, and R2 changes. The same is true when the axial position of the third temperature detection device changes. By changing the position of the temperature detection device, the temperature detection position or the monitored area can be changed to adapt to the size of the lesion site.

To facilitate the movement of the temperature detection device, in one of the embodiments, the radio frequency ablation catheter and the temperature detection device are provided with guiding structures that cooperate with each other therebetween.

For example, in one of the embodiments, the outer wall of the electrode, the sheath or the metal tube is provided with a sliding groove arranged along the axial direction. The temperature detection device surrounds the outer wall of the electrode, the sheath or the metal tube, and a protrusion engaging with the sliding groove is provided on the inner wall of the temperature detection device.

The movement of the temperature detection device is guided by the cooperation of the protrusion and the sliding groove, and thus the rotation thereof relative to the electrode is prevented.

After the temperature detection device is ready, when fixed outside the body, the temperature detection device can be riveted and fixed to the electrode by locally applying a pressure by a plier of corresponding size, or it can be fixed by welding or the like.

If the temperature detection device requires position adjustment in the body, the position of the temperature detection device can be adjusted in a way similar to the pulling of the pull wire 10. For example, in one of the embodiments, a temperature detection device with an adjustable axial position is connected with a pulling string, by means of which the axial position of the temperature detection device relative to the electrode 1 is driven to be changed.

In one of the embodiments, the pulling string extends into the interior of the radio frequency ablation catheter from the connected temperature detection device, and extends towards the proximal end from the interior of the radio frequency ablation catheter.

The interior of the radio frequency ablation catheter may be the interior of the electrode, the sheath or the metal tube. The main portion of the pulling string extends inside the radio frequency ablation catheter to avoid contact with tissues in the body and eliminate the potential risk of cutting the tissues.

The pulling string extends towards the proximal end, that is, extends to approach the operator, thus facilitating control of pulling. The proximal end of the pulling string may be provided with an adjustment component, and the pulling string is driven to move by the manipulation of the adjustment component. In one of the embodiments, the radio frequency ablation catheter further includes a first adjustment component and a second adjustment component able to move relative to each other. The electrode 1 is relatively fixed to the first adjustment component, and the pulling string is connected to the second adjustment component. When the first adjustment component and the second adjustment component move relative to each other, the pulling string drives the connected temperature detection device to change its axial position relative to the electrode 1.

When the first adjustment component and the second adjustment component move relative to each other, the distance between at least a portion of areas of the them will change, either in the axial direction or in the circumferential direction, or with at least a component in a certain direction. Such change in distance drives the pulling string to move the temperature detection device.

In one of the embodiments, the first adjustment component and the second adjustment component are slidably fitted or rotatably fitted.

The slidable fit can be understood as relative movement in the axial direction. The rotatable fit can be understood as relative movement at least in the circumferential direction, for example, rotatable fit with axial position limited or screw-thread rotatable fit.

Since the electrode 1 is relatively fixed to the first adjustment component, the first adjustment component may be combined with the aforementioned first stretch bending component, or even they are the same component. The sheath 2 is fixed at a side of the distal end of the component.

For example, in one of the embodiments, the radio frequency ablation catheter further includes a first stretch bending component 4 and a second stretch bending component 5 that can move relatively close to or away from each other. The electrode 1 is relatively fixed to the first stretch bending component 4, and a pull wire 10 is connected between the second stretch bending component 5 and the electrode.

The radio frequency ablation catheter further includes a first adjustment component and a second adjustment component able to move relative to each other. The electrode 1 is relatively fixed to the first adjustment component, and the pulling string is connected to the second adjustment component. When the first adjustment component and the second adjustment component move relative to each other, the pulling string drives the connected temperature detection device to change its axial position relative to the electrode 1.

The first stretch bending component 4 and the first adjustment component are separately provided or formed as a same component.

Each of the second adjustment component and the second stretch bending component moves independently with respect to first stretch bending component. The movement manner of the second adjustment component and the second stretch bending component are each independent from each other, for example, one is sliding, and the other is rotation.

If the position of the temperature detection device needs to be adjusted in the body, it is possible to mark the displacement of the movement of the pulling string at the proximal end to obtain the position of the temperature detection device relative to the electrode. Alternatively, the temperature detection device is formed by a developing material, and the position of the temperature detection device relative to the electrode can be derived from the image of the temperature detection device.

In one of the embodiments, a pressure sensor 17 is further provided inside the electrode 1, for detecting changes in the contact pressure between the electrode 1 and the tissue ablated.

The pressure sensor 17 is preferably welded and fixed in the electrode 1, and is connected to the connector 7 of the ablation apparatus. When the electrode 1 is brought into contact with the tissue to be ablated and cannot advance further, the pressure sensor 17 detects the pressure change at the electrode 1 and transmits it to the ablation apparatus. Under the guidance by bronchoscopic navigation, the electrode travels through the channel of the bronchoscope, and enter the tissue to be ablated through a hole previously punctured in the bronchial wall near the lesion. In this way, the accurate position of the electrode 1 can be more effectively determined, thereby improving the positioning accuracy of the electrode 1.

Referring to FIG. 8, to install the thermistor 9, the pull wire 10, the pressure sensor 17, and the lead wire 16, the end of the electrode 1 facing the installation direction of the sheath 2 is provided with a first mounting hole 102, a second mounting hole 103, a third mounting hole 104, and a fourth mounting hole 105. The thermistor 9, the pull wire 10, the pressure sensor 17 and the lead wire 16 are installed in the four mounting holes respectively. In addition, a fifth mounting hole may be further provided for connecting the pulling string. When there are multiple pulling strings, mounting holes of corresponding number are provided.

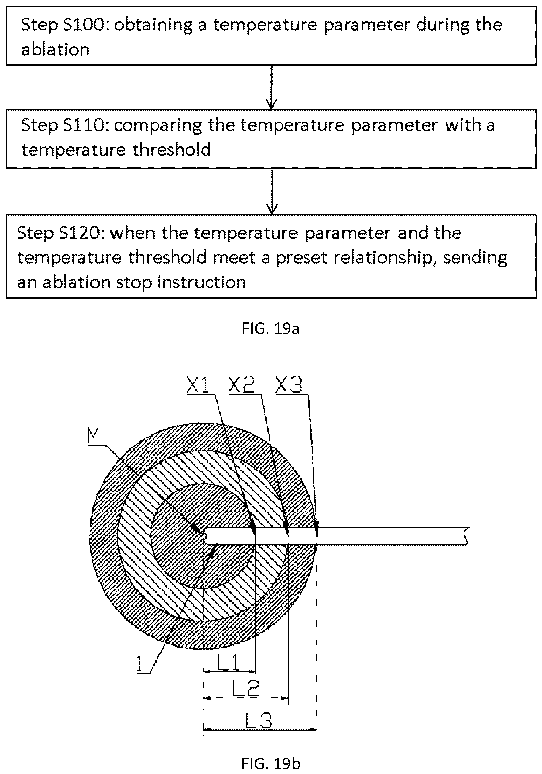

Based on the foregoing embodiments, referring to FIG. 19a, some embodiments of the present application also provide a radio frequency ablation method, which includes
Step S100: obtaining a temperature parameter during the ablation;
Step S110: comparing the temperature parameter with a temperature threshold; and
Step S120: when the temperature parameter and the temperature threshold meet a preset relationship, sending an ablation stop instruction.

For the procedure and specific steps of the radio frequency ablation method, please refer to related descriptions in the control method for radio frequency ablation as below.

Similarly, based on the foregoing implementations, referring to FIG. 19a, some implementations of the present application also provide a temperature monitoring method for temperature monitoring method for radio frequency ablation, which includes
Step S100: obtaining a temperature parameter during the ablation;
Step S110: comparing the temperature parameter with a temperature threshold; and
Step S120: when the temperature parameter and the temperature threshold meet a preset relationship, sending an ablation stop instruction.

Similarly, based on the foregoing implementations, referring to FIG. 19a, some implementations of the present application also provide a control method for radio frequency ablation, which includes
Step S100: obtaining a temperature parameter during the ablation;
Step S110: comparing the temperature parameter with a temperature threshold; and
Step S120: when the temperature parameter and the temperature threshold meet a preset relationship, sending an ablation stop instruction.

Before Step S100, an end portion of a radio frequency ablation catheter is located near the tumor cells or diseased cells, and punctures into the cells. Generally, a distal end portion of an electrode may be made to correspond to the center of a lesion site, and then the electrode is powered to start ablation. The temperature parameter being acquired in real time may be acquired in real time by the aforementioned thermistor 9 or temperature detection device (all of which is also referred to as temperature detection device hereinafter). The specific means of acquiring the temperature may be implemented in combination with other methods in the prior art.

The temperature parameter corresponds spatially a detection site, that is, the location where the temperature detection device locates. Although there are also means or methods for obtaining the temperature parameter in the prior art, most of them are limited to the location of the electrode.

In one of the embodiments, the temperature parameter includes an edge temperature parameter, and the distance between the detection site corresponding to the edge temperature parameter and the distal end portion of the electrode is L, and wherein L0 ≤ L, where L0 is a predicted radius of the lesion site.

The preset relationship includes that the edge temperature parameter reaches the temperature threshold and is maintained for a preset period of time.

In a conventional ablation operation, generally an ablation time is preset, and the ablation is stopped after the time is ended; but the ablation effect cannot be guaranteed. In this embodiment, the ablation effect can be further ensured by determining an ablation end point based on the temperature change at a specified position in the temperature field.

In one of the embodiments, the temperature parameter further includes a first temperature parameter, and the distance between the detection site corresponding to the first temperature parameter and the distal end portion of the electrode is L1, and L1 < L0, where L0 is the predicted radius of the lesion site.

In one of the embodiments, the preset relationship further includes that the first temperature parameter reaches 60°C to 100°C.

The detection site corresponding to the first temperature parameter is inside the lesion site. The ablation process inside the lesion site can be indicated by monitoring the change of the first temperature parameter, for example, whether the ablation process approaches the ablation end point can be determined.

In one of the embodiments, the edge temperature parameter includes a third temperature parameter, and the distance between the detection site corresponding to the third temperature parameter and the distal end portion of the electrode is L3, and L0 < L3, where L0 is the predicted radius of the lesion site.

In the prior art, the radio frequency ablation catheter is guided by B-ultrasound or CT to directly puncture into the lesion site (pathological tissue mass) during ablation. The electrode in the radio frequency ablation catheter is powered to cause the temperature in the tissue to exceed 60°C, resulting in cell death and necrotic areas. If the local tissue temperature exceeds 100°C, the tumor tissue and parenchyma of surrounding organs undergo coagulative necrosis, so a large spherical area of coagulative necrosis is produced during the treatment. There is a further hyperthermia area of 43°C to 60°C outside the area of coagulative necrosis. In this area, cancer cells are killed while normal cells are recoverable.

In one of the embodiments, the preset relationship includes that the third temperature parameter reaches the temperature threshold and is maintained for a preset period of time, where the temperature threshold is 43°C to 60°C, and the period of time is not less than 3 min.

To ensure the ablation effect, for example, the temperature threshold corresponding to the third temperature parameter can be set to be close to or equal to 60°C, such as 55°C to 60°C.

If the third temperature parameter is 43°C to 60°C, it means that the outer edge of the lesion site reaches this temperature. When this temperature is reached, an ablation stop instruction may be sent immediately, or sent after a predetermined time of delay.

In one of the embodiments, the edge temperature parameter further includes a second temperature parameter, and the distance between the detection site corresponding to the second temperature parameter and the distal end portion of the electrode is L2, and L2= L0, where L0 is the predicted radius of the lesion site.

In one of the embodiments, the preset relationship further includes that the second temperature parameter reaches 60°C to 90°C.

The detection site corresponding to the second temperature parameter has substantially the same radius as that of the lesion site, so the temperature at the outer edge of the lesion site can be accurately acquired, which facilitates the accurate temperature monitoring.

In one of the embodiments, the temperature parameter further includes a distal temperature parameter, and the detection site corresponding to the distal temperature parameter is the distal end portion of the electrode.

In one of the embodiments, the preset relationship further includes that the distal temperature parameter reaches 60°C to 100°C.

In one of the embodiments, the temperature parameter includes:
a distal temperature parameter, wherein the detection site corresponding to the distal temperature parameter is the distal end portion of the electrode;
a first temperature parameter, wherein the distance between the detection site corresponding to the first temperature parameter and the distal end portion of the electrode is L1;
a second temperature parameter, wherein the distance between the detection site corresponding to the second temperature parameter and the distal end portion of the electrode is L2; and
a third temperature parameter, wherein the distance between the detection site corresponding to the third temperature parameter and the distal end portion of the electrode is L3,
in which Ll<L0=L2<L3, where L0 is the predicted radius of the lesion site.

By acquiring the temperatures corresponding to multiple sites, the temperature field near the electrode and in the areas around the lesion can be monitored during the ablation process. When the distal end portion of the electrode corresponds to the center of the lesion, the peripheral temperature distribution centered on the lesion as an origin can be obtained, and the ablation process can be indicated according to the change of temperature gradient. The radius of the lesion site may be measured in advance, for example, by an imaging device, and the approximate radius can be calculated. Through the temperature detection at multiple sites, the progress of ablation can be accurately acquired, so as to further guarantee the ablation effect.

In one of the embodiments, the peripheral temperature distribution around the electrode is visually displayed according to the temperature parameter during the ablation process.

During the visualization process, various types of signs or three-dimensional shapes can be used to simulate and display the changes in the temperature field centered on the distal end of the electrode.

Referring to FIG. 19b, Point M is assumed as the distal end portion of the electrode 1, and Point M is the center of the lesion site during the ablation process;
Point M is the distal end portion of the electrode, and the temperature acquired from Point M is the distal temperature parameter;
the distance between Point X1 and Point M is L1, and the temperature acquired from Point X1 is the first temperature parameter;
the distance between Point X2 and Point M is L2, and the temperature acquired from Point X2 is the second temperature parameter;
the radius L0 of the lesion site is equal to L2;
the distance between Point X3 and Point M is L3, and the temperature acquired from Point X3 is the third temperature parameter.

During the ablation process, a schematic diagram of the temperature field as shown in FIG. 19b can be drawn and displayed in real time according to the changes of each temperature parameter. Different cross-sectional areas relates different temperatures, and different colors can be used to distinguish them in actual display. According to the change of temperature, the displayed color is changed accordingly. For example, the color gradually darkens or changes from a cold color to a warm color as the temperature increases.

When the progress of ablation is determined with reference to the schematic diagram of the temperature field, for example:
when the first temperature parameter reaches the temperature threshold, but the second temperature parameter does not reach the temperature threshold, it can be determined that the ablation is ongoing;
when the second temperature parameter reaches the temperature threshold, but the third temperature parameter does not reach the temperature threshold, it can be determined that the ablation is in proximate of being completed; and
when the third temperature parameter reaches the temperature threshold and is maintained for a period of time (usually 3 min), it can be determined that the ablation is completed.
Since the detection site corresponding to the third temperature parameter is in the peripheral area of the lesion, and thus the ablation effect can be ensured by monitoring the ablation end point through the third temperature parameter.

In one of the embodiments, the radio frequency ablation catheter according to the foregoing embodiments is used during radio frequency ablation, and the first temperature parameter, the second temperature parameter, and the third temperature parameter are respectively acquired from the first temperature detection device, the second temperature detection device, and the third temperature detection device. The distal temperature parameter is acquired from the temperature probe mounted at the distal end portion of the electrode.

The radio frequency ablation catheter according to the foregoing embodiments is provided with the temperature probe, the first temperature detection device, the second temperature detection device, and the third temperature detection device.

The temperature probe is mounted at the distal end portion of the electrode, the positions of the remaining three temperature detection devices are arranged in sequence from the distal end to the proximal end, and the distances between the three temperature detection devices and the distal end of the electrode correspond to L1, L2, and L3 respectively.

Since L0 is different in different cases, if needed, the positions of the three-temperature detection device need be adjusted in advance to meet Ll<L0=L2<L3.

For example, with reference to the size of lung tumor cells or diseased cells, which is usually about one centimeter in radius, and certainly taking the temperature conductivity in the body's internal environment into consideration, L3 is set to be 1.5 cm and L1 is set to be one-half of L0 (0.5 cm).

After a period of time of ablation operation, if the temperature parameter has never reached the temperature threshold, the position of the electrode will be adjusted and a second ablation instruction is given. The position of the electrode can be adjusted by means of the pull wire method as described in the above related embodiments, or the radio frequency ablation catheter as a whole is directly advanced or withdrawn, until the temperature parameter reaches the temperature threshold to end the ablation operation.

In each embodiment of the present application, each step in the method flow is not necessarily performed sequentially in the order as described or as shown in the drawings. Unless explicitly stated herein, there is no strict limitation on the order of performing these steps, and these steps can be performed in other orders. Moreover, at least part of the steps can include multiple sub-steps or multiple stages. These sub-steps or stages are not necessarily performed at the same time, but can be performed at different times. The order of performing these sub-steps or stages is not necessarily in sequence, but can be performed alternately with other steps or alternately with at least part of the sub-steps or stages of other steps.

Some implementations of the present application also provide a radio frequency ablation apparatus, which includes
a first module, configured to obtain a temperature parameter during the ablation;
a second module, configured to compare the temperature parameter with a temperature threshold; and
a third module, configured to send an ablation stop instruction when the temperature parameter and the temperature threshold meet a preset relationship.

Similarly, some implementations of the present application also provide a control apparatus for control apparatus for radio frequency ablation, which includes
a first module, configured to obtain a temperature parameter during the ablation;
a second module, configured to compare the temperature parameter with a temperature threshold; and
a third module, configured to send an ablation stop instruction when the temperature parameter and the temperature threshold meet a preset relationship.

Similarly, some implementations of the present application also provide a temperature monitoring apparatus for radio frequency ablation, which includes
a first module, configured to obtain a temperature parameter during the ablation;
a second module, configured to compare the temperature parameter with a temperature threshold; and
a third module, configured to send an ablation stop instruction when the temperature parameter and the temperature threshold meet a preset relationship.

The specific definitions and details of the radio frequency ablation apparatus, the control apparatus for radio frequency ablation, and the temperature monitoring apparatus for radio frequency ablation can be referred to the above definitions for the radio frequency ablation method and the control method for radio frequency ablation, which will not be repeated here again. All or part of the modules in the radio frequency ablation apparatus can be implemented by software, hardware, and a combination thereof. The above modules can be built as hardware in or independent of a processor in computer equipment, or can be stored as software in a memory of the computer equipment, so that the processor can invoke and execute the operations corresponding to the above modules.

Some embodiments of the present application also provide computer equipment, such as a radio frequency ablation apparatus, including a memory and a processor, where a computer program is stored in the memory. When the computer program is executed by the processor, steps of the radio frequency ablation method are performed, including, for example,
Step S 100: obtaining a temperature parameter during the ablation;
Step S 110: comparing the temperature parameter with a temperature threshold; and
Step S 120: when the temperature parameter and the temperature threshold meet a preset relationship, sending an ablation stop instruction.

Some embodiments of the present application also provide computer equipment, such as a temperature monitoring apparatus for radio frequency ablation, including a memory and a processor, wherein a computer program is stored in the memory. When the computer program is executed by the processor, steps of the temperature monitoring method for radio frequency ablation are performed, including, for example,
Step S 100: obtaining a temperature parameter during the ablation;
Step S 110: comparing the temperature parameter with a temperature threshold; and
Step S 120: when the temperature parameter and the temperature threshold meet a preset relationship, sending an ablation stop instruction.

Some embodiments of the present application also provide computer equipment, such as a control apparatus for radio frequency ablation, including a memory and a processor, where a computer program is stored in the memory. When the computer program is executed by the processor, steps of the control method for radio frequency ablation are performed, including, for example,
Step S 100: obtaining a temperature parameter during the ablation;
Step S 110: comparing the temperature parameter with a temperature threshold; and
Step S 120: when the temperature parameter and the temperature threshold meet a preset relationship, sending an ablation stop instruction.

The specific definitions and related details of the radio frequency ablation apparatus, the control apparatus for radio frequency ablation, and the temperature monitoring apparatus for radio frequency ablation, as computer equipment, can be referred to the above definitions for the radio frequency ablation method, the control method for radio frequency ablation, and the temperature monitoring method for radio frequency ablation, which will not be repeated here again.

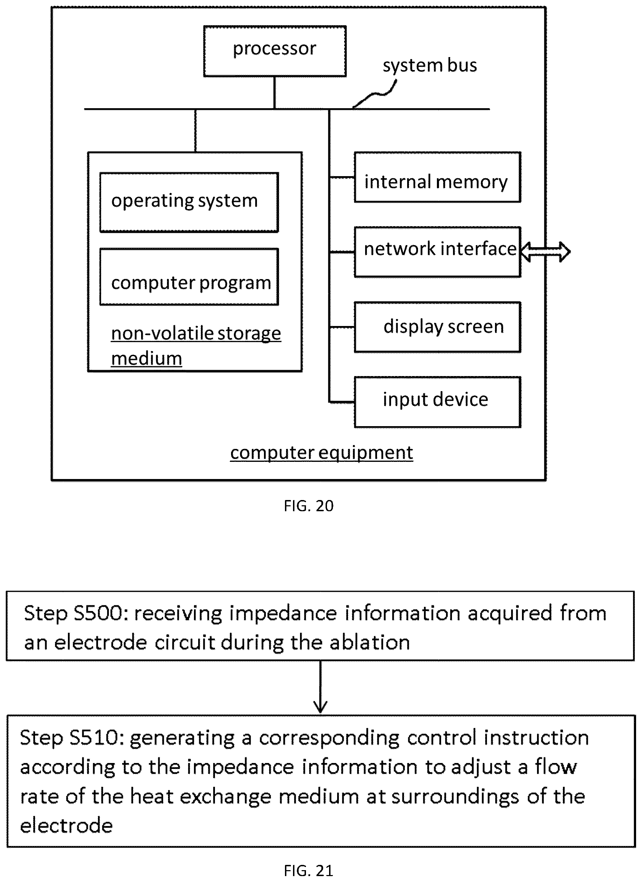

The computer equipment may be a terminal, having an internal structural diagram as shown in FIG. 20. The computer equipment includes a processor, a memory, a network interface, a display screen and an input device connected through a system bus. The processor of the computer equipment is used to provide the calculation and control abilities. The memory of the computer equipment includes a non-volatile storage medium and an internal memory. An operating system and a computer program are stored in the non-volatile storage medium. The internal memory provides an environment for the operation of the operating system and computer program in the non-volatile storage medium. The network interface of the computer equipment is used to communicate with an external terminal through network connection. The computer program is executed by the processor to perform the radio frequency ablation method, the control method for radio frequency ablation, and the temperature monitoring method for radio frequency ablation. The display screen of the computer equipment may be a liquid crystal display screen or an E-ink display screen. The input device of the computer equipment may be a touch layer covered on the display screen, a button, a trackball or a touchpad provided on the housing of the computer equipment, or an externally connected keyboard, touchpad, or mouse.

Those skilled in the art can understand that the structure shown in FIG. 20 is only a block diagram of part of the structure related to the solution of the present application, and does not constitute a limitation on the computer equipment to which the solution of the present application is applicable. The specific computer equipment may include more or fewer elements than those shown in the figure, or some of the elements, or have a different arrangement of elements.

In one embodiment, a computer-readable storage medium is provided, in which a computer program is stored. When the computer program is executed by a processor, steps of the radio frequency ablation method are performed, including, for example,
Step S 100: obtaining a temperature parameter during the ablation;
Step S 110: comparing the temperature parameter with a temperature threshold; and
Step S120: when the temperature parameter and the temperature threshold meet a preset relationship, sending an ablation stop instruction.

In one embodiment, a computer-readable storage medium is provided, in which a computer program is stored. When the computer program is executed by a processor, steps of the temperature monitoring method for radio frequency ablation are performed, including, for example,
Step S 100: obtaining a temperature parameter during the ablation;
Step S 110: comparing the temperature parameter with a temperature threshold; and
Step S120: when the temperature parameter and the temperature threshold meet a preset relationship, sending an ablation stop instruction.

In one embodiment, a computer-readable storage medium is provided, in which a computer program is stored. When the computer program is executed by a processor, steps of the control method for radio frequency ablation are performed, including, for example,
Step S 100: obtaining a temperature parameter during the ablation;
Step S 110: comparing the temperature parameter with a temperature threshold; and
Step S120: when the temperature parameter and the temperature threshold meet a preset relationship, sending an ablation stop instruction.

Other specific definitions and related details can be referred to the above definitions for the radio frequency ablation method and the control method for radio frequency ablation, which will not be repeated here again.

Persons of ordinary skill in the art can understand that all or part of the procedures of the methods in the above-mentioned methods can be implemented by relevant hardware under instruction of a computer program. The computer program may be stored in a non-volatile computer readable storage medium; and when the computer program is executed, the procedures of the methods in above-mentioned embodiments are implemented. Any reference to the memory, storage, database or other media used in the embodiments provided in this application includes non-volatile and/or volatile memory. Non-volatile memory may include read only memory (ROM), programmable ROM (PROM), electrically programmable ROM (EPROM), electrically erasable programmable ROM (EEPROM), or flash memory. Volatile memory may include random access memory (RAM) or external cache memory. For the purpose of illustration but not limitation, RAM is available in many forms, such as static RAM (SRAM), dynamic RAM (DRAM), synchronous DRAM (SDRAM), double data rate SDRAM (DDRSDRAM), enhanced SDRAM (ESDRAM), Synchlink DRAM (SLDRAM), Rambus direct RAM (RDRAM), direct Rambus dynamic RAM (DRDRAM), and Rambus dynamic RAM (RDRAM).

The radio frequency ablation catheters according to the above embodiments are preferably applied in the radio frequency ablation of lungs. That is, it can be understood that in the present invention, a radio frequency ablation catheter for lungs, corresponding ablation method and apparatus, corresponding ablation control method and control apparatus, and corresponding temperature monitoring method and temperature monitoring apparatus are preferably provided.

In one of the embodiments, the present application also provides a radio frequency ablation system for lungs, In the radio frequency ablation system for lungs, the radio frequency ablation catheter according to the above embodiments can be and is preferably used. When the radio frequency ablation catheter according to the above embodiments is used, the relevant structural features and method flow can be referred to the above embodiments, which will not be repeated in this embodiment.

A radio frequency ablation system for lungs include:
a radio frequency ablation catheter, which may be the radio frequency ablation catheter according to the above embodiments;
a heat exchange medium delivery apparatus, configured to provide a heat exchange medium to the surroundings of an electrode in the radio frequency ablation catheter; and
a control module, configured to drive the heat exchange medium delivery apparatus according to impedance information in a circuit where the electrode of the radio frequency ablation catheter is located.

After the electrode in the radio frequency ablation catheter is powered, the surrounding temperature increases. To avoid drying and charring of the surrounding tissues, a heat exchange medium is used for protection. The real-time output of the heat exchange medium can be adjusted according to the change of the impedance information of the electrode circuit. When the electrode is working, a circuit is formed, and the measurement and calculation of the impedance in the circuit can be implemented as described in the prior art.

If an electrode driving signal (such as, a voltage signal applied) is controlled according to only the temperature, there will generally be a delay in signal feedback, because the conduction of temperature is related to the body's internal environment and the thermal conductivity of related devices. In this embodiment, a corresponding control logic relationship is established between the output amount of the heat exchange medium and the impedance information. The impedance information is a more direct electrical signal, which is advantageous to the real-time monitoring, so as to avoid the delay.

Because of the timely feedback of impedance information, stable output of the electrode can be maintained, without frequently adjusting the electrode driving signal according to the temperature in the lesion site, whereby the ablation process is stable and easy to control.

In a preferred embodiment, the electrode driving signal remains unchanged during the ablation process.

The electrode driving signal comes from the ablation apparatus and is transmitted to the electrode through a circuit. The electrode driving signal can be that known in the prior art, which is not the focus of improvement in this application.

In one of the embodiments, the radio frequency ablation system for lungs further includes a temperature detection device for acquiring temperature information in surrounding areas of the electrode, and the control module is further configured to prompt or control the ablation process according to the temperature information.

The temperature in the surrounding areas of the electrode reflects the ablation process to a certain extent. For example, the lesion site can be inactivated after being heated for a period of time at a certain temperature. At this time, the control module may output a prompt instruction or output an ablation stop instruction to stop the working of the electrode. For example, when the temperature is too high, the output of the heat exchange medium may be abnormal, and the control module can output a prompt instruction to give an alarm.

In one of the embodiments, one temperature detection device is provided, or multiple temperature detection devices are provided. The distance from the position of at least one temperature detection device i.e., the temperature information acquisition point, to the electrode is 0.5 cm to 3 cm, preferably 1 cm to2 cm, for example 1.5 cm.

With reference to the aforementioned related embodiments, since the electrode is generally located in the central area of the lesion site during operation, at least one temperature information acquisition point needs to be spaced from the electrode for a certain distance, so as to suggest the ablation process in a space with the distance as a radius.

The surrounding area of the electrode is a three-dimensional space centered at the electrode. The impedance sensor and the temperature detection device may be configured separately, but are preferably integrally mounted on the radio frequency ablation catheter to maintain a relative position with respect to the electrode.

In one of the embodiments, the control module drives the heat exchange medium delivery apparatus to adjust the flow rate of the heat exchange medium. The heat exchange medium delivery apparatus includes conventional fluid delivery devices such as containers, pipelines, pumps, valves, and flow meters. The delivery volume of the heat exchange medium can be adjusted by at least the devices such as controlled valves or pumps. The delivery volume of the heat exchange medium is adjusted by the control module according to a corresponding control instruction. After the flow rate of the heat exchange medium is changed, the acquired impedance information changes accordingly.

To maintain a stable ablation process, in one of the embodiments, the control module compares the impedance information with a threshold, and makes the impedance information approach a steady-state impedance by adjusting the flow rate of the heat exchange medium.

To make the impedance information approach the threshold, the two are constantly compared according to a certain sampling period during operation, and the flow rate of the heat exchange medium is adjusted correspondingly according to the relationship of magnitude between the two to realize closed-loop control.

The threshold can be a numerical point or a numerical range. When it is a numerical range, the impedance information is compared with the upper and lower limits of the numerical range, so that the impedance information is within the numerical range.

Due to the different internal environments or lesion sites in different patients, in one of the embodiments, the steady-state impedance is first determined in the initial stage, and the threshold is calculated based on the steady-state impedance.

The impedance information may be an impedance value or other parameters related to the impedance value, and the threshold has a corresponding form with it to facilitate the comparison therebetween. For example, if the impedance value is higher than the threshold, it means that the flow rate of the heat exchange medium needs to be increased to further improve the infiltration or cooling effect, and vice versa.

In one of the embodiments, the steady-state impedance is determined by outputting the heat exchange medium at an initial flow rate after the radio frequency ablation catheter is in place in the body before the electrode is powered, and acquiring the impedance information in real time, where after the impedance information becomes stable, a corresponding value is recorded as steady-state impedance.

After the radio frequency ablation catheter is in place in the body, the initial flow rate of the heat exchange medium (for example, 0.5 ml/min) can be determined according to experiences or the historical data. At this stage, due to the perfusion of the heat exchange medium, the impedance information will fluctuate, for example the perfusion of physiological saline may cause the impedance information to decrease. The impedance information is acquired in real time. When the impedance information does not decrease any longer and remains stable, the impedance information at this time is the steady-state impedance.

When the threshold is a numerical range, the upper and lower limits of the threshold are the upper and lower impedances respectively. The upper and lower impedances can be calculated with reference to the steady-state impedance, and even the steady-state impedance is used. For example, the lower impedance may be the steady-state impedance. A specific calculation method of the upper impedance and the lower impedance can be determined according to empirical data or the patient's personal condition.

In one of the embodiments, the threshold is a numerical range. In the process of adjusting the flow rate of the heat exchange medium, the control module also acquires the impedance information in real time, determines the change trend of the impedance information, and accordingly changes the adjustment amplitude of the flow rate of the heat exchange medium according to the change trend of the resistance information or compares the impedance information with one of the upper and lower limits of the threshold.

The flow rate of the heat exchange medium is generally adjusted at intervals by a certain step size. Whether maintaining the current flow rate or further changing the flow rate can be determined by the change trend of the impedance information. The change trend of the impedance information may be increasing or decreasing. For example, when the flow rate of the heat exchange medium is increased, the intended objective is to reduce the impedance information. If the change trend of the impedance information is still increasing, then the flow rate of the heat exchange medium can be further increased. That is, relative to the initial flow rate of the heat exchange medium, the magnitude of adjustment is further increased.

When the change trend of the impedance information is decreasing, it indicates that the intended objective is achieved. At this time, the acquired impedance information can be directly compared with the lower limit of the threshold to determine whether the flow rate of the heat exchange medium needs to be reduced.

Referring to FIG. 21, based on the aforementioned radio frequency ablation system for lungs, in one of the embodiments, a radio frequency ablation method for lungs is further provided, which includes
Step S500: receiving impedance information acquired from an electrode circuit during the ablation; and
Step S510: generating a corresponding control instruction based on the impedance information to adjust the flow rate of the heat exchange medium at the surroundings of the electrode.

For the specific process and details of the radio frequency ablation method for lungs, please refer to the following descriptions of a control method for radio frequency ablation for lungs.

Referring also to FIG. 21, based on the aforementioned radio frequency ablation system for lungs, in one of the embodiments, an impedance monitoring method for radio frequency ablation for lungs is further provided, which includes
Step S500: receiving impedance information acquired from an electrode circuit during the ablation; and
Step S510: generating a corresponding control instruction based on the impedance information to adjust the flow rate of the heat exchange medium at the surroundings of the electrode.

Referring also to FIG. 21, based on the aforementioned radio frequency ablation system for lungs, in one of the embodiments, a control method for radio frequency ablation for lungs is further provided, which includes
Step S500: receiving impedance information acquired from an electrode circuit during the ablation; and
Step S510: generating a corresponding control instruction based on the impedance information to adjust the flow rate of the heat exchange medium at the surroundings of the electrode.

The ablation, the acquisition of impedance information, and the output of the heat exchange medium can all be implemented in connection with the radio frequency ablation catheter or related equipment and systems in the previous embodiments, and the related specific structures will not be repeated here again.

In one of the embodiments, Step S500 also includes predetermining a steady-state impedance, and calculating a threshold according to the steady-state impedance. The threshold is compared with the impedance information in Step S510 to generate a corresponding control instruction.

In one of the embodiments, the steady-state impedance is determined by outputting the heat exchange medium at an initial flow rate after the radio frequency ablation catheter is in place in the body before the electrode is powered, and acquiring the impedance information in real time, wherein after the impedance information becomes stable, a corresponding value is recorded as steady-state impedance.

In Step S510, the generating a corresponding control instruction based on the impedance information specifically includes:
Step S511: comparing the impedance information with the threshold, and determining whether the flow rate is to be increased or decreased based on the relationship between the impedance information and the threshold; and
Step S512: generating a corresponding control instruction according to a predetermined magnitude of increase or decrease based on the determined increase or decrease of the flow rate.

Determining increase or decrease of the flow rate is to be understood as the intended change or demand of the flow rate, for example, further increasing the flow rate based on the current flow rate, or further reducing the flow rate based on the current flow rate. The magnitude of increase or decrease can be made by a preset step size.

For example, the current flow rate is X ml/s. When the flow rate needs to be further increased, by sending a control instruction to the heat exchange medium delivery apparatus, the flow rate becomes X+Y ml/s, wherein Y can be deemed as an increase amplitude. The same applies when the flow rate needs to be further reduced.

In one of the embodiments, the amplitudes of increase and decrease are each independently a fixed value or a dynamic value.

The increase amplitude or decrease amplitude can be a fixed value, or a dynamic value, for example, in relation to the current impedance information; or a difference between the impedance information and the threshold is set as Z, and the increase or decrease is a dynamic value and is related to Z. For example, the closer the current impedance information is to the threshold, the smaller the increase amplitude or decrease amplitude will be, so that the control is more precise and the feedback delay is minimized.

The relationship between the impedance information and the threshold may be a simple numerical ratio, or satisfy other functional relationships. This depends on the specific physical parameters, change forms and even units of measurement selected for the impedance information and the threshold.

In one of the embodiments, the impedance information and the threshold are both impedance values in ohms. This facilitates the comparison. With regard to the measurement and calculation of the impedance value, if the threshold is a numerical range, in Step S511, the determining whether the flow rate is to be increased or decreased based on the relationship between the impedance information and the threshold includes specifically:
the flow rate being determined to be increased when the impedance information is greater than the upper limit of the threshold;
the flow rate being determined to be decreased when the impedance information is less than the lower limit of the threshold; and
maintaining the current flow rate, when the impedance information is within the threshold range,
wherein in Step S511, when the flow rate is determined to be increased, a first control instruction is generated in Step S512, wherein the flow rate of the heat exchange medium corresponding to the first control instruction is greater than the current flow rate; and
in Step S511, when the flow rate is determined to be decreased, a second control instruction is generated in Step S512, wherein the flow rate of the heat exchange medium corresponding to the second control instruction is less than the current flow rate.

The control instruction as an electrical signal can directly change the rotation speed of a pump or the opening of a valve, etc. The actions of these controlled devices also reflect the changes in the flow rate of the heat exchange medium. Therefore, the form of the control instruction is not strictly limited, provided that it can at least correspond to the change in the flow rate of the heat exchange medium.

The impedance information is acquired in real time, or may be understood as being acquired following a predetermined sampling period and constantly compared with the threshold. These operations run throughout the ablation process. That is, Step S500 and Step S510 are performed cyclically.

In one of the embodiments, Step S500 and Step S510 are cyclically performed according to the sampling period of the impedance information, wherein
after a control instruction is generated and output in a previous sampling period, in a next period, after the impedance information is acquired, it is first compared with the impedance information in the previous sampling period before comparison with the threshold, to determine the change trend of the impedance information; and
according to the change trend of the impedance information, the adjustment magnitude of the flow rate of the heat exchange medium is correspondingly changed or the impedance information is compared with one of the upper or lower limits of the threshold.

The control instruction in the previous sampling period may be the first control instruction or the second control instruction. For different types of control instructions, different means of determination can be used in the next period.

For example, in one of the embodiments, after the first control instruction is generated and output in a previous sampling period, in a next period, after the impedance information is acquired, it is first compared with the impedance information in the previous sampling period before comparison with the threshold, to determine the change trend of the impedance information, wherein
when the change trend of the impedance information is increasing, the adjustment amplitude of the flow rate of the heat exchange medium is increased; and
when the change trend of the impedance information is decreasing, the impedance information in the current sampling period is compared with the lower limit of the threshold.

For example, in one of the embodiments, after the second control instruction is generated and output in a previous sampling period, in a next period, after the impedance information is acquired, it is first compared with the impedance information in the previous sampling period before comparison with the threshold, to determine the change trend of the impedance information, wherein
when the change trend of the impedance information is decreasing, the adjustment amplitude of the flow rate of the heat exchange medium is increased; and
when the change trend of the impedance information is increasing, the impedance information in the current sampling period is compared with the upper limit of the threshold.

The end of the ablation process may be based on a preset time or based on the temperature of the electrode or the lesion site. In one of the embodiments, it also includes prompting or controlling the ablation process by the radio frequency ablation method, the control method, or the impedance monitoring method in the foregoing embodiments.

For example, the following steps are also performed while the impedance information is monitored in real time:
Step S 100: obtaining a temperature parameter during the ablation;
Step S 110: comparing the temperature parameter with a temperature threshold; and
Step S 120: when the temperature parameter and the temperature threshold meet a preset relationship, sending an ablation stop instruction. The specific steps for temperature monitoring can be made reference to the aforementioned related embodiments.

In this embodiment, the temperature parameter acquired from surrounding areas of the electrode is received in real time during the ablation process, and the ablation process is prompted or controlled according to the temperature parameter.

The temperature in the surrounding areas of the electrode reflects the ablation process to a certain extent. For example, the lesion site can be inactivated after being heated for a period of time at a certain temperature. At this time, the control module can output a prompt instruction or output an ablation stop instruction to stop the working of the electrode. For example, when the temperature is too high, the output of the heat exchange medium may be abnormal, and the control module can output a prompt instruction to give an alarm.

In one of the embodiments, the distance between the acquisition point of the temperature parameter and the electrode is 0.5 cm to 3 cm. After the temperature parameter reaches 43°C to60°C and is maintained for a preset time, an ablation stop instruction is sent.

The ablation stop instruction may be to directly disconnect the electrode power supply, or give prompt information. Use of the temperature parameter to prompt or control the ablation process is logically independent of use of the impedance information to control the flow rate of the heat exchange medium. Use of the impedance information to control the flow rate of the heat exchange medium focuses on the regulation of the ablation process, and use of the temperature parameter to prompt or control the ablation process is only to intervene at important process nodes, for example, by disconnecting the circuit or popping up a prompt when Step S500 and Step S510 are performed cyclically.

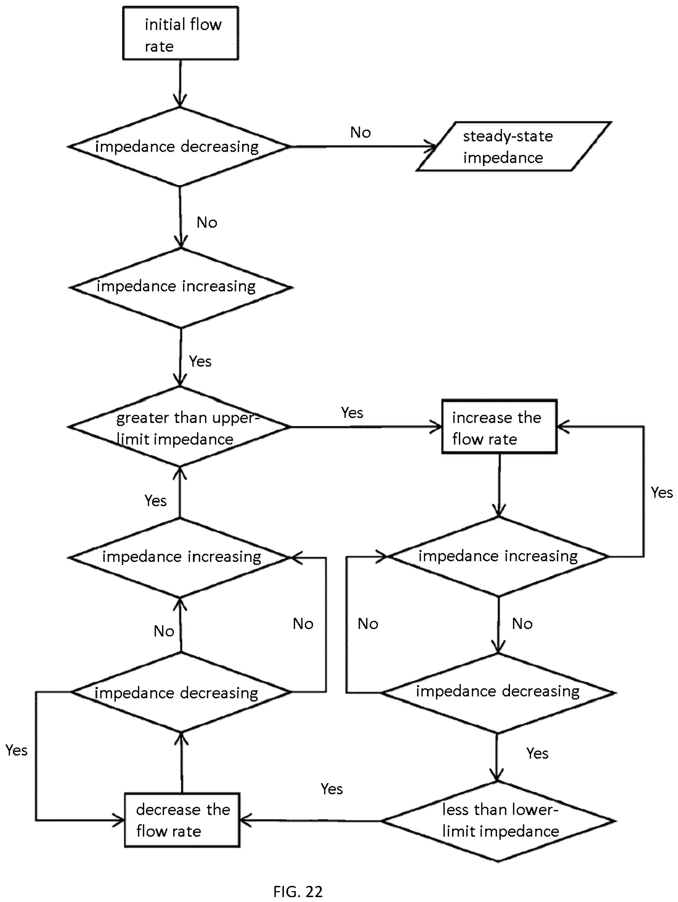

Referring to FIG. 22, in one of the embodiments, a process of a radio frequency ablation method or control method for lungs includes perfusing the heat exchange medium at an initial flow rate, to allow the heat exchange medium to flow out through the infiltration cover outside the electrode to form a protective layer, and acquiring and monitoring the impedance change during the process, wherein the current impedance is the steady-state impedance when the impedance is stable.

The electrode is powered to start ablation, and the impedance information is acquired in real time. The obtained impedance information is constantly compared with a threshold, and a corresponding control instruction is generated to adjust the flow rate of the heat exchange medium. The threshold is a numerical range, and the lower limit impedance can be the steady state impedance.

As the ablation progresses, the impedance increases. When the impedance is greater than the set upper limit impedance, a first control instruction is generated and sent, that is, to increase the flow rate of the heat exchange medium.

Then the impedance information in a next sampling period is compared with the impedance information in the previous sampling period to determine whether the impedance is increasing. When it is still increasing, the first control instruction is generated and sent again, that is, to further increase the flow rate of the heat exchange medium.

When the impedance is no longer increasing but starts to decrease, it is compared with the lower limit impedance. When the impedance is less than the preset lower limit impedance, a second control instruction is generated and sent, that is, to decrease the flow rate of the heat exchange medium.

Then the impedance information in a next sampling period is compared with the impedance information of the previous sampling period to determine whether the impedance is decreasing. When it is still decreasing, the second control instruction is generated and sent again, that is, to further decrease the flow rate of the heat exchange medium until the impedance starts to increase.

Based on the above cycle, the impedance information is constantly monitored and the flow rate of the heat exchange medium is adjusted during the ablation process. When a preset condition is met, for example, by calculating the time or monitoring the temperature, the ablation status is prompted or the ablation is terminated.

Each step in the radio frequency ablation method for lungs and the control method for radio frequency ablation for lungs is not necessarily performed sequentially in the order as described or as shown in the drawings. Unless explicitly stated herein, there is no strict limitation on the order of performing these steps, and these steps can be performed in other orders. Moreover, at least part of the steps can include multiple sub-steps or multiple stages. These sub-steps or stages are not necessarily performed at the same time, but can be performed at different times. The order of performing these sub-steps or stages is not necessarily in sequence, but can be performed alternately with other steps or alternately with at least part of the sub-steps or stages of other Steps.

Based on the aforementioned radio frequency ablation method for lungs, in this embodiment, a radio frequency ablation apparatus for lungs is provided, which includes
an acquisition module, configured to receive impedance information acquired from an electrode circuit during the ablation; and
an adjustment module, configured to generate a corresponding control instruction according to the impedance information to adjust the flow rate of a heat exchange medium at the surrounding of the electrode.

Similarly, based on the aforementioned impedance monitoring method for radio frequency ablation for lungs, in this embodiment, an impedance monitoring apparatus for radio frequency ablation for lungs is provided, which includes:
an acquisition module, configured to receive impedance information acquired from an electrode circuit during the ablation; and
an adjustment module, configured to generate a corresponding control instruction according to the impedance information to adjust the flow rate of a heat exchange medium at the surroundings of the electrode.

Similarly, based on the aforementioned control method for radio frequency ablation for lungs, in this embodiment, a control apparatus for radio frequency ablation for lungs is provided, which includes
an acquisition module, configured to receive impedance information acquired from an electrode circuit during the ablation; and
an adjustment module, configured to generate a corresponding control instruction according to the impedance information to adjust the flow rate of a heat exchange medium at the surroundings of the electrode.

The specific definitions and related details of the radio frequency ablation apparatus for lungs, the control apparatus for radio frequency ablation for lungs, and the impedance monitoring apparatus for radio frequency ablation for lungs according to the embodiments can be respectively referred to the above definitions for the radio frequency ablation method for lungs, the control method for radio frequency ablation for lungs, and the impedance monitoring method for radio frequency ablation for lungs, which will not be repeated here again. All or part of the modules in the radio frequency ablation apparatus for lungs and the control apparatus for radio frequency ablation for lungs can be implemented by software, hardware, and a combination thereof. The above modules can be built as hardware in or independent of a processor in computer equipment, or can be stored as software in a memory of the computer equipment, so that the processor can invoke and execute the operations corresponding to the above modules.

Some embodiments of the present application also provide computer equipment, such as a radio frequency ablation apparatus for lungs, including a memory and a processor, wherein a computer program is stored in the memory. When the computer program is executed by the processor, steps of the radio frequency ablation method for lungs are performed, including, for example,
Step S500: receiving impedance information acquired from an electrode circuit during the ablation; and
Step S510: generating a corresponding control instruction according to the impedance information to adjust the flow rate of the heat exchange medium at the surroundings of the electrode.

Some embodiments of the present application also provide computer equipment, such as an impedance monitoring apparatus for radio frequency ablation for lungs, including a memory and a processor, wherein a computer program is stored in the memory. When the computer program is executed by the processor, steps of the impedance monitoring method for radio frequency ablation for lungs are performed, including, for example,
Step S500: receiving impedance information acquired from an electrode circuit during the ablation; and
Step S510: generating a corresponding control instruction according to the impedance information to adjust the flow rate of the heat exchange medium at the surroundings of the electrode.

Some embodiments of the present application also provide computer equipment, such as a control device for radio frequency ablation for lungs, including a memory and a processor, wherein a computer program is stored in the memory. When the computer program is executed by the processor, steps of the control method for radio frequency ablation for lungs are performed, including, for example, Step S500: receiving impedance information acquired from an electrode circuit during the ablation; and Step S510: generating a corresponding control instruction according to the impedance information to adjust the flow rate of the heat exchange medium at the surroundings of the electrode.

The specific definitions and related details of the radio frequency ablation apparatus for lungs and the control apparatus for radio frequency ablation for lungs, as computer equipment, can be referred to the above definitions for the radio frequency ablation method for lungs and the control method for radio frequency ablation for lungs, which will not be repeated here again.

The radio frequency ablation apparatus for lungs and the control apparatus for radio frequency ablation for lungs, as computer equipment, may be terminals, having an internal structural diagram as shown in FIG. 20. The computer equipment includes a processor, a memory, a network interface, a display screen and an input device connected through a system bus. The processor of the computer equipment is used to provide the calculation and control abilities. The memory of the computer equipment includes a non-volatile storage medium and an internal memory. An operating system and a computer program are stored in the non-volatile storage medium. The internal memory provides an environment for the operation of the operating system and computer program in the non-volatile storage medium. The network interface of the computer equipment is used to communicate with an external terminal through network connection. The computer program is executed by the processor to perform the radio frequency ablation method or the control method for radio frequency ablation. The display screen of the computer equipment may be a liquid crystal display screen or an E-ink display screen, and the input device of the computer equipment may be a touch layer covered on the display screen, a button, a trackball or a touchpad provided on the housing of the computer equipment, or an externally connected keyboard, touchpad, or mouse.

Those skilled in the art can understand that the structure shown in FIG. 20 is only a block diagram of part of the structure related to the solution of the present application, and does not constitute a limitation on the computer equipment to which the solution of the present application is applicable. The specific computer equipment may include more or fewer members than shown in the figure or include combinations of some members, or have a different arrangement of members.

In one embodiment, a computer-readable storage medium is provided, in which a computer program is stored. When the computer program is executed by a processor, steps of the radio frequency ablation method for lungs are performed, including, for example,
Step S500: receiving impedance information acquired from an electrode circuit during the ablation; and
Step S510: generating a corresponding control instruction according to the impedance information to adjust the flow rate of the heat exchange medium at the surroundings of the electrode.

In one embodiment, a computer-readable storage medium is provided, in which a computer program is stored. When the computer program is executed by a processor, steps of the impedance monitoring method for radio frequency ablation for lungs are performed, including, for example,
Step S500: receiving impedance information acquired from an electrode circuit during the ablation; and
Step S510: generating a corresponding control instruction according to the impedance information to adjust the flow rate of the heat exchange medium at the surroundings of the electrode.

In one embodiment, a computer-readable storage medium is provided, in which a computer program is stored. When the computer program is executed by a processor, steps of the control method for radio frequency ablation for lungs are performed, including, for example,
Step S500: receiving impedance information acquired from an electrode circuit during the ablation; and
Step S510: generating a corresponding control instruction according to the impedance information to adjust the flow rate of the heat exchange medium at the surroundings of the electrode.

Other specific definitions and related details can be referred to the above definitions for the radio frequency ablation method for lungs and the control method for radio frequency ablation for lungs, which will not be repeated here again.

Persons of ordinary skill in the art can understand that all or part of the procedures of the methods in the above-mentioned methods can be implemented by relevant hardware under instruction of a computer program. The computer program may be stored in a non-volatile computer readable storage medium; and when the computer program is executed, the procedures of the methods in above-mentioned embodiments are implemented. Any reference to the memory, storage, database or other media used in the embodiments provided in this application includes non-volatile and/or volatile memory. Non-volatile memory may include read only memory (ROM), programmable ROM (PROM), electrically programmable ROM (EPROM), electrically erasable programmable ROM (EEPROM), or flash memory. Volatile memory may include random access memory (RAM) or external cache memory. For the purpose of illustration but not limitation, RAM is available in many forms, such as static RAM (SRAM), dynamic RAM (DRAM), synchronous DRAM (SDRAM), double data rate SDRAM (DDRSDRAM), enhanced SDRAM (ESDRAM), Synchlink DRAM (SLDRAM), Rambus direct RAM(RDRAM), direct Rambus dynamic RAM (DRDRAM), and Rambus dynamic RAM(RDRAM).

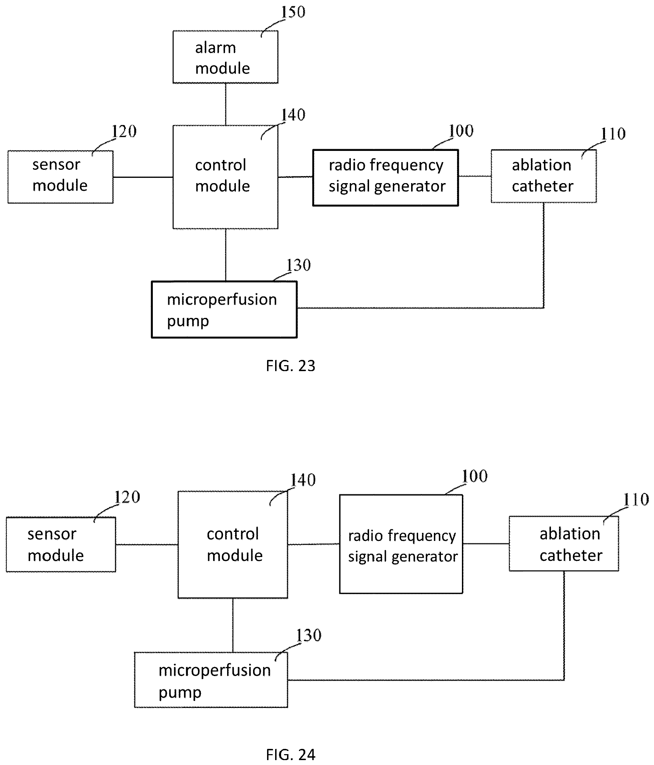

In one of the embodiments of the present application, a radio frequency ablation system for lungs is further provided. Referring to FIG. 23, in this embodiment, the radio frequency ablation system for lungs includes a radio frequency signal generator 100, an ablation catheter 110, an electrode pad, a sensor module 120, a microperfusion pump 130, a control module 140 and an alarm module 150.

In this embodiment, the radio frequency signal generator 100 can be the aforementioned ablation apparatus (or a part of the ablation apparatus); the ablation catheter 110 can be the radio frequency ablation catheter according to various embodiments above; the heat exchange medium is specifically, for example, physiological saline, and the heat exchange medium flow channel is a transmission channel for liquid transmission; the microperfusion pump 130 can be contemplated as one means or device of the heat exchange medium delivery apparatus; and the control module 140 can be the radio frequency ablation apparatus according to each embodiment above.

In addition, the control module 140, the alarm module 150, and the micro-perfusion pump 130 can be partially or completely integrated in the spatial arrangement, for example, integrated into the ablation apparatus to form a complete ablation apparatus.

In this embodiment, the radio frequency signal generator 100 is connected to the control module 140, and configured to receive a command from the control module 140 to generate a radio frequency signal and transmit the radio frequency signal to the ablation catheter 110.

In this embodiment, the ablation catheter 110 has an electrical transmission channel for electrical transmission and a transmission channel for liquid transmission. The radio frequency signal generator 100 is connected to the electrical transmission channel, and the liquid transmission channel is connected to the microperfusion pump 130.

The ablation catheter 110 can receive the radio frequency signal generated by the radio frequency signal generator 100, and exert the radio frequency signal onto a tissue to be ablated after punctured into the tissue to be ablated; and can further receive the physiological saline perfused by the microperfusion pump 130. A front end of the ablation catheter 110, i.e., the end that extends into the human body, is provided with a liquid outlet hole, which is configured to perfuse the physiological saline into the tissue to be ablated after punctured into the tissue to be ablated.

In the foregoing related embodiments, the infiltration cover 20 is mounted around the electrode 1, and a plurality of dispersing holes 200 are uniformly arranged on the infiltration cover 20. When the infiltration cover 20 is provided, the liquid outlet hole mentioned in this embodiment may be deemed as a dispersing hole 200.

In other embodiments, the ablation catheter 110 may be provided with other structures, as long as the physiological saline can be perfused into the tissue to be ablated. For example, the ablation catheter 110 has a bendable structure that can be controlled to bend the front end of the ablation catheter 110, so as to accurately transfer the front end of the ablation catheter 110 to the site to be treated; and/or, an operating unit for controlling the bendable structure may be provided outside the ablation catheter for operation by a medical personnel to control the bending degree of the bendable structure.

Specifically, the ablation catheter 110 is guided by B-ultrasound or CT to travel through a bronchoscope and puncture into the tissue to be ablated via a puncture point, and transfers the radio frequency signal to the tissue to be ablated. At this time, the electrode pad connected to the radio frequency signal generator 100 and attached to the patient's body is conducted to the ablation catheter 110 to form an electric field. The tissue to be ablated is also in the electric field, and a high-frequency current acts on the human tissue therebetween, causing the coagulation, denaturation, and necrosis of tumor cells in contact with the electrode at the distal end of the radio frequency ablation catheter. The ablation catheter 110 is only an electrode that transfers energy, and the temperature increase is caused by the high-speed oscillations and friction with each other of ions and thus the conversion of radio frequency energy into heat energy in the tumor tissue near the ablation catheter 110 after a circuit is formed. In other words, the ablation catheter 110 is passively heated due to the elevated temperature of nearby tissues. After the tissue is energized, it becomes dry and charred, forming a "scab" around the ablation catheter 110 that "vacuum encloses" the ablation catheter 110. The impedance between the ablation catheter 110 and the "scab" tissue will instantly sharply increase (the thermal precipitation and high impedance effects during radio frequency ablation), causing the ablation to stop, and resulting in incomplete ablation and insufficient range of ablation. In this embodiment, the ablation catheter 110 is provided with a liquid outlet hole to perfuse the physiological saline into the tissue to be ablated. Since the physiological saline is conductive, after the tissue to be ablated is infiltrated, the impedance is ensured to be stabilized in a certain range throughout the entire ablation process, so that the radio frequency energy can be continuously output. In this way, a large enough range of ablation is formed to produce a larger and more effective coagulative necrosis.

In this embodiment, the sensor module 120 is provided on the ablation catheter 110, and the sensor module 120 is connected to the control module 140. Specifically, the sensor module 120 is provided at one end of the ablation catheter 110 that is in contact with the tissue to be ablated. The sensor module 120 includes an impedance sensor and a temperature sensor, which are configured to detect the impedance and the temperature at the contact position of the ablation catheter 110 with the tissue to be ablated and send the temperature information and impedance information to the control module 140. Specifically, the temperature sensor may be a thermocouple.

The temperature sensor may also be the thermistor 9, the electrode ring 8, or the temperature detection device configured on the radio frequency ablation catheter according to the foregoing embodiments. The impedance sensor can be understood as, for example, in the form of a sampling circuit, which is configured to obtain the impedance information in the electrode circuit.

In this embodiment, the impedance information is acquired by the impedance sensor and transmitted to the control module 140. In other embodiments, the impedance information may be calculated based on the voltage and current values acquired by the system. Specifically, real-time voltage and current values are measured by a voltage and current measuring device and transmitted to the control module 140, and the impedance is calculated by the control module 140 based on the real-time voltage and current values. In other embodiments, the sensor module 120 may further include a flow sensor, a pressure sensor, and other types of sensors, which are configured to detect the flow rate of the physiological saline, the pressure at the contact position of the ablation catheter 110 with the tissue to be ablated, and other data, to monitor the ablation process, so as to respond in time when abnormalities occur. For example, when flow information of the physiological saline transmitted by the flow sensor is lower than a certain threshold, the microperfusion pump 130 is controlled by the control module 140 to increase the amount of perfusion of the physiological saline to avoid the "scabbing" of human tissue. In other embodiments, the type and number of sensors in the sensor module can be set according to the requirements and actual situations during operation of the radio frequency ablation system for lungs to monitor the ablation process.

In this embodiment, the microperfusion pump 130 is connected to the control module 140 and the ablation catheter 110, and configured to receive a command from the control module 140 to perfuse physiological saline to the ablation catheter 110. In other embodiments, the microperfusion pump 130 may receive a command from the control module 140 to perfuse other liquids into the ablation catheter 110, as long as the electrical and thermal conductivity of the tissue to be ablated are improved, balance of the impedance is maintained, the impedance is maintained in a relatively stable state, the temperature of the tissue to be ablated is decreased, and the moisture content in the tissue to be ablated is increased, so as to fundamentally prevent the tissue to be ablated from scabbing due to drying and heating while no serious side effects occur to the human body.

In this embodiment, the alarm module 150 is connected to the control module 140, and configured to receive an alarm command sent by the control module 140 and give an alarm.

In this embodiment, the control module 140 is connected to the radio frequency signal generator 100, the sensor module 120, the microperfusion pump 130, and the alarm module 150. Specifically, the control module 140 is configured to control the radio frequency signal generator 100 to generate a radio frequency signal. Specifically, the control module 140 receives the impedance information and temperature information detected by the sensor module 120, and controls the microperfusion pump 130 to perfuse physiological saline to the ablation catheter 110 based on the impedance information. In this embodiment, when the impedance sensor detects a sharp increase in the impedance, it means that the tissue near the electrode of the ablation catheter 110 is being dried and charred, which will cause scabs. After the control module 140 receives the impedance information from the impedance sensor, it controls the microperfusion pump 130 to increase the amount of perfusion of physiological saline. In this embodiment, the control module 140 is also configured to determine whether the perfusion of the physiological saline is smooth based on the temperature information, and to give an alarm command if not. Specifically, if the temperature is found to increase to exceed a certain threshold (for example, 85°C) during the ablation process, the control module 140 determines that the perfusion of the physiological saline is blocked, and gives an alarm command to control the alarm module 150 to give an alarm signal for prompt, thereby ensuring the smooth progress of the ablation process.

The alarming regarding the extreme conditions during the ablation process can be made by temperature monitoring, impedance information monitoring, or a combination thereof. The alarming is implemented or the ablation is stopped once one of them extends beyond an acceptable upper limit.

In other embodiments, when the sensor module 120 further includes a flow sensor, a pressure sensor, and other types of sensors, the control module 140 further receives other data information sent by the sensor module 120 to monitor the ablation process, and gives a control command in time for processing when abnormalities occur. In other embodiments, the control module 140 can make a comprehensive judgment on the ablation process based on the data transmitted by multiple sensors, and control the microperfusion pump 130, the radio frequency signal generator 100 and other devices for adjustment when preset conditions are met, to ensure the smooth progress of the ablation process. The preset conditions can be set by a user according to the actual situations during operation of the radio frequency ablation system for lungs and the setting of the sensors. In other embodiments, the impedance information may be calculated based on the voltage and current values acquired by the system. Specifically, real-time voltage and current values are measured by a voltage and current measuring device and transmitted to the control module 140, and the impedance is calculated by the control module 140 based on the real-time voltage and current values.

The electrode pad is connected to the radio frequency signal generator and configured to form a circuit together with the electrode in the ablation catheter through the human body.

The electrode pad is attached to a suitable site on the patient's body. During ablation, the electrode in the ablation catheter forms a working circuit with the electrode pad through the human body (that is, the circuit where the electrode is located), and a high-frequency current acts on the human tissue therebetween, causing the coagulation, denaturation, and necrosis of the lesion site in contact with the electrode. The electrode pad itself is a conventional element in the ablation operation and is not shown in the figure.

In this embodiment, once the radio frequency ablation system for lungs starts to operate, the control module 140 controls the microperfusion pump 130 to perfuse physiological saline to the ablation catheter 110. After receiving the information transmitted from the impedance sensor that the impedance increases sharply, the control module 140 controls the microperfusion pump 130 to increase the amount of perfusion of the physiological saline. In other embodiments, at the time when the radio frequency ablation system for lungs starts to operate, the microperfusion pump 130 does not perfuse physiological saline. Only after receiving the information transmitted from the impedance sensor that the impedance increases sharply, the control module 140 controls the microperfusion pump 130 to perfuse physiological saline to the ablation catheter 110.

FIG. 24 is a schematic diagram showing a radio frequency ablation system for lungs according to another embodiment of the present invention.

In this embodiment, the radio frequency ablation system for lungs includes a radio frequency signal generator 100, an ablation catheter 110, an electrode pad, a sensor module 120, a microperfusion pump 130 and a control module 140.

In this embodiment, the radio frequency signal generator 100 is connected to the control module 140, and configured to receive an instruction from the control module 140 to generate a radio frequency signal and transmit the radio frequency signal to the ablation catheter 110.

In this embodiment, the ablation catheter 110 has an electrical transmission channel for electrical transmission and a transmission channel for liquid transmission. The radio frequency signal generator 100 is connected to the electrical transmission channel, and the liquid transmission channel is connected to the microperfusion pump 130. The ablation catheter 110 can receive the radio frequency signal generated by the radio frequency signal generator 100, and exert the radio frequency signal onto a tissue to be ablated after punctured into the tissue to be ablated; and can further receive the physiological saline perfused by the microperfusion pump 130. A front end of the ablation catheter 110, i.e., the end that extends into the human body, is provided with a liquid outlet hole, which is configured to perfuse the physiological saline into the tissue to be ablated after punctured into the tissue to be ablated. In other embodiments, the ablation catheter 110 may be provided with other structures, as long as the physiological saline can be perfused into the tissue to be ablated. For example, the ablation catheter 110 has a bendable structure that can be controlled to bend the front end of the ablation catheter 110, so as to accurately transfer the front end of the ablation catheter 110 to the site to be treated; and/or, an operating unit for controlling the bendable structure may be provided outside the ablation catheter for operation by a medical personnel to control the bending degree of the bendable structure.

Specifically, the ablation catheter 110 is guided by B-ultrasound or CT to travel through a bronchoscope and puncture into the tissue to be ablated via a puncture point, and transfers the radio frequency signal to the tissue to be ablated. At this time, the electrode pad connected to the radio frequency signal generator 100 and attached to the patient's body is conducted to the ablation catheter 110 to form an electric field. The tissue to be ablated is also in the electric field, and a high-frequency current acts on the human tissue therebetween, causing the coagulation, denaturation, and necrosis of tumor cells in contact with the electrode at the distal end of the radio frequency ablation catheter. The ablation catheter 110 is only an electrode that transfers energy, and the temperature increase is caused by the high-speed oscillations and friction with each other of ions and thus the conversion of radio frequency energy into heat energy in the tumor tissue near the ablation catheter 110 after a circuit is formed. In other words, the ablation catheter 110 is passively heated due to the elevated temperature of nearby tissues. After the tissue is energized, it becomes dry and charred, forming a "scab" around the ablation catheter 110 that "vacuum wraps" the ablation catheter 110. The impedance between the ablation catheter 110 and the "scab" tissue will instantly sharply increase (the thermal precipitation and high impedance effects during radio frequency ablation), causing the ablation to stop, and resulting in incomplete ablation and insufficient range of ablation. In this embodiment, the ablation catheter 110 is provided with a liquid outlet hole to perfuse the physiological saline into the tissue to be ablated. Since the physiological saline is conductive, after the tissue to be ablated is infiltrated, the impedance is ensured to be stabilized in a certain range throughout the entire ablation process, so that the radio frequency energy can be continuously output. In this way, a large enough range of ablation is formed to produce a larger and more effective coagulative necrosis.

In this embodiment, the sensor module 120 is provided on the ablation catheter 110, and the sensor module 120 is connected to the control module 140. Specifically, the sensor module 120 is provided at one end of the ablation catheter 110 that is in contact with the tissue to be ablated. The sensor module 120 includes an impedance sensor and a temperature sensor, which are configured to detect the impedance and the temperature at the contact position of the ablation catheter 110 with the tissue to be ablated and send the temperature information and impedance information to the control module 140. Specifically, the temperature sensor may be a thermocouple. In this embodiment, the impedance information is acquired and transmitted to the control module 140 by the impedance sensor. In other embodiments, the impedance information may be calculated based on the voltage and current values acquired by the system. Specifically, real-time voltage and current values are measured and transmitted to the control module 140 by a voltage and current measuring device, and the impedance is calculated by the control module 140 based on the real-time voltage and current values. In other embodiments, the sensor module 120 may further include a flow sensor, a pressure sensor, and other types of sensors, which are configured to detect the flow rate of the physiological saline, the pressure at the contact position of the ablation catheter 110 with the tissue to be ablated, and other data, to monitor the ablation process, so as to respond in time when abnormalities occur. For example, when flow information of the physiological saline transmitted by the flow sensor is lower than a certain threshold, the microperfusion pump 130 is controlled by the control module 140 to increase the amount of perfusion of the physiological saline to avoid the "scabbing" of human tissue. In other embodiments, the type and number of sensors in the sensor module can be set according to the requirements and actual situations during operation of the radio frequency ablation system for lungs to monitor the ablation process.

In this embodiment, the microperfusion pump 130 is connected to the control module 140 and the ablation catheter 110, and configured to receive a command from the control module 140 to perfuse physiological saline to the ablation catheter 110. In other embodiments, the microperfusion pump 130 may receive a command from the control module 140 to perfuse other liquids into the ablation catheter 110, as long as the electrical and thermal conductivity of the tissue to be ablated are improved, balance of the impedance is maintained, the impedance is maintained in a relatively stable state, the temperature of the tissue to be ablated is decreased, and the humidity of the tissue to be ablated is increased, so as to fundamentally prevent the tissue to be ablated from scabbing due to drying and heating while no serious side effects occur to the human body.

In this embodiment, the control module 140 is connected to the radio frequency signal generator 100, the sensor module 120 and the microperfusion pump 130. Specifically, the control module 140 is configured to control the radio frequency signal generator 100 to generate a radio frequency signal. Specifically, the control module 140 receives the impedance information and temperature information detected by the sensor module 120, and controls the microperfusion pump 130 to perfuse physiological saline to the ablation catheter 110 based on the impedance information. In this embodiment, when the impedance sensor detects a sharp increase of the impedance, it means that the tissue near the electrode of the ablation catheter 110 is being dried and charred, which will cause scabs. After the control module 140 receives the impedance information from the impedance sensor, it controls the microperfusion pump 130 to increase the amount of perfusion of the physiological saline. In this embodiment, the control module 140 is also configured to determine whether the perfusion of the physiological saline is smooth based on the temperature information, so as to monitor the ablation process. In other embodiments, the control module 140 can make a comprehensive judgment on the ablation process based on the data transmitted by multiple sensors, and control the microperfusion pump 130, the radio frequency signal generator 100 and other devices for adjustment when preset conditions are met, to ensure the smooth progress of the ablation process. The preset conditions can be set by a user according to the actual situations during operation of the radio frequency ablation system for lungs and the setting of the sensors. In other embodiments, the impedance information may be calculated based on the voltage and current values acquired by the system. Specifically, real-time voltage and current values are measured and transmitted to the control module 140 by a voltage and current measuring device, and the impedance is calculated by the control module 140 based on the real-time voltage and current values.

The electrode pad is connected to the radio frequency signal generator and configured to form a circuit together with the electrode in the ablation catheter through the human body.

The electrode pad is attached to a suitable site on the patient's body. During ablation, the electrode in the ablation catheter forms a working circuit with the electrode pad through the human body (that is, the circuit where the electrode is located), and a high-frequency current acts on the human tissue therebetween, causing the coagulation, denaturation, and necrosis of the lesion site in contact with the electrode. The electrode pad itself is a conventional element in the ablation operation and is not shown in the figure.

In this embodiment, once the radio frequency ablation system for lungs starts to operate, the control module 140 controls the microperfusion pump 130 to perfuse physiological saline to the ablation catheter 110. After receiving the information transmitted from the impedance sensor that the impedance increases sharply, the control module 140 controls the microperfusion pump 130 to increase the amount of perfusion of the physiological saline. In other embodiments, at the time when the radio frequency ablation system for lungs starts to operate, the microperfusion pump 130 does not perfuse physiological saline. Only after receiving the information transmitted from the impedance sensor that the impedance increases sharply, the control module 140 controls the microperfusion pump 130 to perfuse physiological saline to the ablation catheter 110.

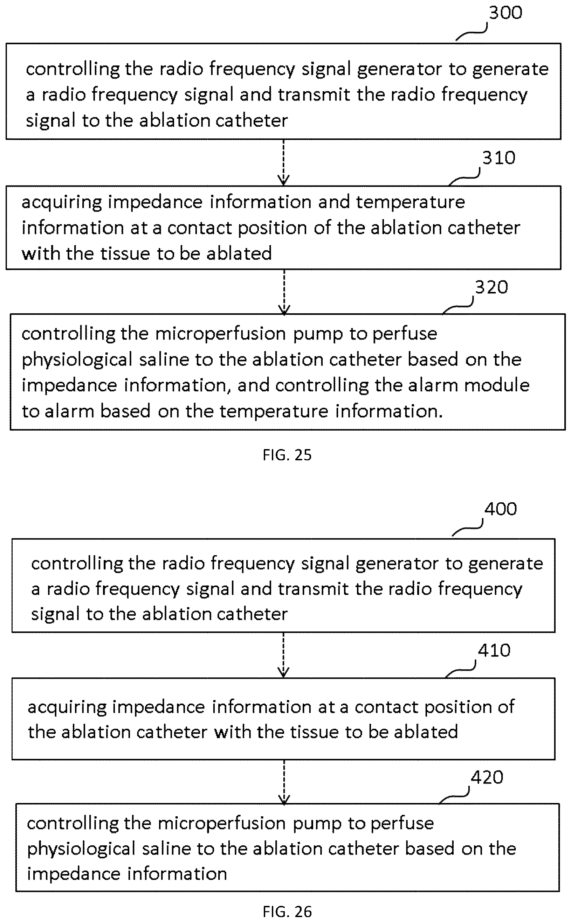

Referring to FIGs. 25 and 26, a radio frequency ablation method for lungs is further provided in embodiments of the present application, which can be implemented by related members, apparatuses, and systems according to the aforementioned embodiments.

The radio frequency ablation method for lungs includes:
controlling the radio frequency signal generator to generate a radio frequency signal and transmitting the radio frequency signal to the ablation catheter;
acquiring impedance information and temperature information at a contact position of the ablation catheter with the tissue to be ablated; and
controlling the microperfusion pump to perfuse physiological saline to the ablation catheter based on the impedance information, and controlling the alarm module to alarm based on the temperature information.

For the specific process and details of the radio frequency ablation method for lungs, please refer to the following description of a control method for radio frequency ablation for lungs.

Referring to FIGs. 25 and 26, a temperature and impedance monitoring method for radio frequency ablation for lungs is further provided in embodiments of the present application, which can be implemented by related members, apparatuses, and systems according to the aforementioned embodiments.

The temperature and impedance monitoring method for radio frequency ablation for lungs includes:
controlling the radio frequency signal generator to generate a radio frequency signal and transmitting the radio frequency signal to the ablation catheter;
acquiring impedance information and temperature information at a contact position of the ablation catheter with the tissue to be ablated; and
controlling the microperfusion pump to perfuse physiological saline to the ablation catheter based on the impedance information, and controlling the alarm module to alarm based on the temperature information.

For the specific process and details of the temperature and impedance monitoring method for radio frequency ablation for lungs, please refer to the following description of a control method for radio frequency ablation for lungs.

FIG. 25 is a schematic diagram showing a control method for radio frequency ablation for lungs according to an embodiment of the present invention.

In this embodiment, the control method for radio frequency ablation for lungs includes:
Step 300: controlling the radio frequency signal generator to generate a radio frequency signal and transmit the radio frequency signal to the ablation catheter.

Specifically, the control module controls the radio frequency signal generator to generate a radio frequency signal, and transmit the radio frequency signal to the ablation catheter. After the ablation catheter is punctured into the tissue to be ablated, the radio frequency signal is transferred to the tissue to be ablated, and the radio frequency signal is converted into heat energy in the circuit, which acts on the tissue to be ablated to cause the coagulation, denaturation, and necrosis of tumor cells in contact with the electrode at the distal end of the ablation catheter.

Step 310: acquiring impedance information and temperature information at a contact position of the ablation catheter with the tissue to be ablated.

Specifically, during the radio frequency ablation, the heat generated causes the temperature of human tissues to increase, causing the human tissues near the ablation catheter to become dry and charred to form "scabs". The resistance between the electrode and "scabs" increases sharply, causing the ablation to stop, and resulting in insufficient ablation. At this time, the impedance sensor detects a sharp increase in the impedance, and transmits the impedance information to the control module. The temperature sensor detects temperature information and transmits it to the control module.

In other embodiments, the impedance information may be calculated based on the voltage and current values acquired by the system. Specifically, real-time voltage and current values are measured and transmitted to the control module by a voltage and current measuring device, and the impedance is calculated by the control module based on the real-time voltage and current values.

Step 320: controlling the microperfusion pump to perfuse physiological saline to the ablation catheter based on the impedance information, and controlling the alarm module to alarm based on the temperature information.

Specifically, after the control module receives the information transmitted from the impedance sensor that the impedance increases sharply, the control module controls the microperfusion pump to increase the amount of perfusion of the physiological saline. The physiological saline is perfused into the ablation catheter and into the tissue to be ablated through the liquid outlet hole on the ablation catheter, to improve the electrical and thermal conductivity of the tissue, thus maintaining balance of the impedance, maintaining the impedance in a relatively stable state, reducing the temperature and increasing the moisture content of the tissue. This fundamentally prevents the tissue from scabbing due to the drying and heating, allows the impedance to be stabilized in a certain range throughout the entire ablation process, and enables the continuous output of radio frequency energy, thereby forming a large enough range of ablation to cause larger and more effective coagulative necrosis of the lesion. In addition, the control module also receives temperature information transmitted from the temperature sensor. When the temperature is found to increase to exceed a certain threshold during the ablation process, the control module determines that the perfusion of the physiological saline is blocked, and gives an alarm command to control the alarm module to give an alarm signal for prompt, thereby ensuring the smooth progress of the ablation process.

FIG. 26 is a schematic diagram showing a control method for radio frequency ablation for lungs according to another embodiment of the present invention.

In this embodiment, the control method for radio frequency ablation for lungs includes:
Step 400: controlling the radio frequency signal generator to generate a radio frequency signal and transmit the radio frequency signal to the ablation catheter.

Specifically, the control module controls the radio frequency signal generator to generate a radio frequency signal, and transmit the radio frequency signal to the ablation catheter. After the ablation catheter is punctured into the tissue to be ablated, the radio frequency signal is transferred to the tissue to be ablated, and the radio frequency signal is converted into heat energy in the circuit, which acts on the tissue to be ablated, causing the coagulation, denaturation, and necrosis of tumor cells in contact with the electrode at the distal end of the ablation catheter.

Step 410: acquiring impedance information at a contact position of the ablation catheter with the tissue to be ablated.

Specifically, during the radio frequency ablation, the heat generated causes the temperature of human tissues to increase, causing the human tissues near the ablation catheter to become dry and charred to form "scabs". The resistance between the electrode and "scabs" increases sharply, causing the ablation to stop, and resulting in insufficient ablation. At this time, the impedance sensor detects a sharp increase in the impedance, and transmits the impedance information to the control module.

In other embodiments, the impedance information may be calculated based on the voltage and current values acquired by the system. Specifically, real-time voltage and current values are measured and transmitted to the control module by a voltage and current measuring device, and the impedance is calculated by the control module based on the real-time voltage and current values.

Step 420: controlling the microperfusion pump to perfuse physiological saline to the ablation catheter based on the impedance information.

Specifically, after the control module receives the information transmitted from the impedance sensor that the impedance increases sharply, the control module controls the microperfusion pump to increase the amount of perfusion of the physiological saline. The physiological saline is perfused into the ablation catheter and into the tissue to be ablated through the liquid outlet hole on the ablation catheter, to improve the electrical and thermal conductivity of the tissue, thus maintaining balance of the impedance, maintaining the impedance in a relatively stable state, reducing the temperature and increasing the moisture content of the tissue. This fundamentally prevents the tissue from scabbing due to the drying and heating, allows the impedance to be stabilized in a certain range throughout the entire ablation process, and enables the continuous output of radio frequency energy, thereby forming a large enough range of ablation to cause larger and more effective coagulative necrosis of the lesion.

Exemplarily, during the operation of the radio frequency ablation system for lungs, the control module controls the radio frequency signal generator to generate a radio frequency signal, and transmit the radio frequency signal to the ablation catheter. After the ablation catheter is punctured into the tissue to be ablated, the radio frequency signal is transferred to the tissue to be ablated, and the radio frequency signal is converted into heat energy in the circuit, which acts on the tissue to be ablated to cause the coagulation, denaturation, and necrosis of tumor cells in contact with the electrode at the distal end of the ablation catheter. During the ablation, the heat generated causes the temperature of human tissues to increase, causing the human tissues near the ablation catheter to become dry and charred to form "scabs". The resistance between the electrode and "scabs" increases sharply, causing the ablation to stop, and resulting in incomplete ablation. At this time, the impedance sensor detects a sharp increase in the impedance, and transmits the impedance information to the control module. After the control module receives the impedance information transmitted from the impedance sensor, or after the impedance is calculated by the control module based on the real-time voltage and current information, the control module controls the microperfusion pump to increase the amount of perfusion of the physiological saline. The physiological saline is perfused into the ablation catheter and into the tissue to be ablated through the liquid outlet hole on the ablation catheter, to improve the electrical and thermal conductivity of the tissue, thus maintaining balance of the impedance, maintaining the impedance in a relatively stable state, reducing the temperature and increasing the moisture content of the tissue. This fundamentally prevents the tissue from scabbing due to the drying and heating, allows the impedance to be stabilized in a certain range throughout the entire ablation process, and enables the continuous output of radio frequency energy, thereby forming a large enough range of ablation to cause larger and more effective coagulative necrosis of the lesion. In addition, the control module also receives temperature information transmitted from the temperature sensor. When the temperature is found to increase to exceed a certain threshold during the ablation process, the control module determines that the perfusion of the physiological saline is blocked, and gives an alarm command to control the alarm module to give an alarm signal for prompt, thereby ensuring the smooth progress of the ablation process.

In the radio frequency ablation system and control method for lungs, the change in impedance of the tissue to be ablated is detected by the impedance sensor. When the impedance is detected to increase sharply, it means that the tissue to be ablated near the electrode is being dried and charred, which will cause scabs. At this time, the physiological saline perfused into the tissue to be ablated is controlled, to reduce the temperature and increase the moisture content of the tissue, thereby fundamentally preventing the tissue from scabbing due to the drying and heating. Moreover, the physiological saline can improve the electrical and thermal conductivity of the tissue, maintaining balance of the impedance, and maintaining the impedance in a relatively stable state. Taken together, the impedance is ensured to be stabilized in a certain range throughout the entire ablation process, so that the radio frequency energy can be continuously output. In this way, a large enough range of ablation is formed to produce a larger and more effective coagulative necrosis.

The technical features of the above-described embodiments may be arbitrarily combined. For the sake of brevity of description, not all possible combinations of the technical features in the above embodiments are described. However, where no contradiction exists, all the combinations of these technical features are contemplated in the scope of the present application.

The above-described embodiments are merely illustrative of several implementations of the present invention, and the description is specific and particular, but is not to be construed as limiting the scope of the present application. It should be pointed out that for those of ordinary skill in the art, several variations and improvements can be made without departing from the concept of the present application, which are all regarded as falling within the protection scope of the present application. Therefore, the protection scope of the present application is defined by the appended claims.

## Claims

1. A radio frequency ablation catheter, comprising an electrode (1), wherein a heat exchange medium flow channel is defined inside the electrode (1); the electrode (1) is provided with a dispersing device; at least one dispersing hole (200) is provided on the dispersing device and communicated with the heat exchange medium flow channel; and a heat exchange medium output from the heat exchange medium flow channel is distributed by and flows out of the dispersing device;
wherein a plurality of dispersing holes (200) are provided for forming a uniform heat exchange medium protective layer on an outer periphery of the electrode (1);
**characterized in that** the dispersing device and the electrode (1) are separately formed, an outflow hole (106) communicating with the heat exchange medium flow channel is defined on an outer wall of the electrode (1), the dispersing device is an infiltration cover (20) mounted on the electrode (1) surrounding the outflow hole (106), a gap is defined between an inner peripheral wall of the infiltration cover (20) and the outer peripheral wall of the electrode (1), and the dispersing holes (200) are defined on the infiltration cover (20), and wherein the heat exchange medium output from the outflow hole (106) flows to the gap between the electrode (1) and the infiltration cover (20), and then flows out from the dispersing holes (200), forming a water layer on an outer surface of the infiltration cover (20).

2. The radio frequency ablation catheter according to claim 1, wherein the heat exchange medium flow channel comprises a main flow channel (115) and a plurality of branch flow channels (116) communicating with the main flow channel (115), and wherein an end of each branch flow channel (116) extends to an outer surface of the electrode (1).

3. The radio frequency ablation catheter according to claim 1, wherein the infiltration cover (20) is fixed to the electrode (1), rotatably mounted on the electrode (1) about the axis of the electrode (1), or slidably mounted on the electrode (1) along the axial direction of the electrode (1).

4. The radio frequency ablation catheter according to claim 1, wherein a plurality of infiltration covers (20) are mounted on the electrode (1), and each infiltration cover (20) moves independently with respect to the electrode (1) or at least two infiltration covers (20) move in association with each other.

5. The radio frequency ablation catheter according to claim 1, wherein the infiltration cover (20) is of a cylindrical structure which is circumferentially closed, and is mounted around the outer periphery of the electrode (1), wherein the number of the dispersing holes (200) is greater than the number of the outflow hole (106).

6. The radio frequency ablation catheter according to claim 1, wherein at least a part of the infiltration cover (20) is formed as a permeable area where the dispersing holes (200) are distributed, and an area of the electrode (1) where the outflow hole (106) is defined corresponds to the permeable area, with the gap defined between the area and an inner wall of the permeable area.

7. The radio frequency ablation catheter according to claim 6, wherein the dispersing holes (200, 201, 202) and the outflow hole (106) are arranged in an offset manner.

8. The radio frequency ablation catheter according to claim 6, wherein the outer wall of the electrode (1) is provided with a recessed area (107), the outflow hole (106) is arranged in the recessed area (107), the permeable area is surrounding the recessed area (107), and the gap is defined between the inner wall of the infiltration cover (20) and a surface of the recessed area (107).

9. The radio frequency ablation catheter according to claim 8, wherein the outflow hole (106) is flared, and the flared area serves as the recessed area (107); and the gap between the inner wall of the infiltration cover (20) and the surface of the recessed area (107) decreases as the distance from the outflow hole (106) increases.

10. The radio frequency ablation catheter according to claim 8, wherein the radio frequency ablation catheter has an additional feature a), or additional features a) and b):
a) the gap between the inner wall of the infiltration cover (20) and the surface of the recessed area (107) increases as the distance from the outflow hole (106) increases;
b) one recessed area (107) is provided or more recessed areas (107) isolated from each other are provided, with one outflow hole (106) is provided in one recessed area (107); and wherein within one recessed area (107), the gap between the inner wall of the infiltration cover (20) and the surface of the recessed area (107) increases as the distance from the outflow hole (106) in the recessed area increases.

11. The radio frequency ablation catheter according to claim 8, wherein the radio frequency ablation catheter has an additional feature c), or additional features c) and d):
c) the recessed area (107) is a distribution groove extending along the axial direction of the electrode (1), several groups of outflow holes (106) are arranged in a circumferential direction of the electrode (1), and each group of outflow holes (106) corresponds to a respective distribution groove;
d) one outflow hole (106) is defined at a bottom of each distribution groove, and the depth of the distribution groove increases as the distance from the outflow hole (106) increases.

12. The radio frequency ablation catheter according to claim 1, wherein the diameter of the dispersing hole (200, 201, 202) increases as the distance from the outflow hole (106) increases.

13. A radio frequency ablation system for lungs, comprising:
a radio frequency ablation catheter according to any one of claims 1 to 12;
a heat exchange medium delivery apparatus, configured to provide a heat exchange medium to surroundings of an electrode (1) of the radio frequency ablation catheter; and
a control module (140), configured to drive the heat exchange medium delivery apparatus accordingly based on impedance information in a circuit where the electrode (1) of the radio frequency ablation catheter is located.

14. The radio frequency ablation system for lungs according to claim 13, further comprising a temperature detection device for acquiring temperature information in a surrounding area of the electrode (1), wherein the control module (140) is further configured to prompt or control the ablation process according to the temperature information.

15. The radio frequency ablation system for lungs according to claim 14, wherein one or more temperature detection device are provided, and a distance from a position of at least one of the one or more temperature detection devices to the electrode (1) is 0.5 cm to 3 cm.

## Patentansprüche

1. Radiofrequenzablationskatheter, umfassend:
eine Elektrode (1), wobei
innerhalb der Elektrode (1) ein Wärmeaustauschmedium-Strömungskanal definiert ist; wobei die Elektrode (1) mit einer Dispersionsvorrichtung versehen ist;
wobei
mindestens eine Dispersionsöffnung (200) an der Dispersionsvorrichtung vorgesehen ist und mit dem Wärmeaustauschmedium-Strömungskanal verbunden ist; und
wobei ein Wärmeaustauschmedium, das von dem Wärmeaustauschmedium-Strömungskanal austritt, durch die Dispersionsvorrichtung verteilt wird und aus dieser herausfließt; wobei
mehrere Dispersionsöffnungen (200) vorgesehen sind, um eine gleichmäßige Wärmeaustauschmedium-Schutzschicht auf einem Außenumfang der Elektrode (1) zu bilden;
**dadurch gekennzeichnet, dass**
die Dispersionsvorrichtung und die Elektrode (1) getrennt ausgebildet sind,
wobei
ein mit dem Wärmeaustauschmedium-Strömungskanal in Verbindung stehendes Ausflussloch (106) an einer Außenwand der Elektrode (1) definiert ist,
wobei
die Dispersionsvorrichtung eine an der Elektrode (1) angebrachte Infiltrationsabdeckung (20) ist, die das Ausflussloch (106) umgibt, wobei
ein Zwischenraum zwischen einer inneren Umfangswand der Infiltrationsabdeckung (20) und der äußeren Umfangswand der Elektrode (1) definiert ist, und wobei
die Dispersionsöffnungen (200) auf der Infiltrationsabdeckung (20) definiert sind, und wobei
das aus dem Ausflussloch (106) austretende Wärmeaustauschmedium zu dem Zwischenraum zwischen der Elektrode (1) und der Infiltrationsabdeckung (20) fließt und dann aus den Dispersionsöffnungen (200) herausfließt, wobei
es eine Wasserschicht auf einer äußeren Oberfläche der Infiltrationsabdeckung (20) bildet.

2. Radiofrequenzablationskatheter gemäß Anspruch 1, wobei
der Wärmeaustauschmedium-Strömungskanal einen Hauptströmungskanal (115) und mehrere Zweigströmungskanäle (116) umfasst, die mit dem Hauptströmungskanal (115) verbunden sind, und wobei
sich ein Ende jedes Zweigströmungskanals (116) zu einer äußeren Oberfläche der Elektrode (1) hin erstreckt.

3. Radiofrequenzablationskatheter gemäß Anspruch 1, wobei
die Infiltrationsabdeckung (20) an der Elektrode (1) angebracht ist, drehbar an der Elektrode (1) um die Achse der Elektrode (1) gehalten wird oder entlang der axialen Richtung der Elektrode (1) verschiebbar an der Elektrode (1) angeordnet ist.

4. Radiofrequenzablationskatheter gemäß Anspruch 1, wobei
mehrere Infiltrationsabdeckungen (20) an der Elektrode (1) gehalten werden und jede Infiltrationsabdeckung (20) sich unabhängig hinsichtlich der Elektrode (1) bewegt oder mindestens zwei Infiltrationsabdeckungen (20) sich gemeinsam bewegen.

5. Radiofrequenzablationskatheter gemäß Anspruch 1, wobei
die Infiltrationsabdeckung (20) eine zylindrische Struktur aufweist, die in Umfangsrichtung geschlossen ist, und um den Außenumfang der Elektrode (1) herum gehalten wird, wobei
die Anzahl der Dispersionsöffnungen (200) größer ist als die Anzahl der Ausflusslöcher (106).

6. Radiofrequenzablationskatheter gemäß Anspruch 1, wobei
zumindest ein Teil der Infiltrationsabdeckung (20) als ein durchlässiger Bereich ausgebildet ist, in dem die Dispersionsöffnungen (200) verteilt sind, und ein Bereich der Elektrode (1), in dem das Ausflussloch (106) definiert ist, dem durchlässigen Bereich entspricht, wobei
der Zwischenraum zwischen dem Bereich und einer Innenwand des durchlässigen Bereichs definiert ist.

7. Radiofrequenzablationskatheter gemäß Anspruch 6, wobei
die Dispersionsöffnungen (200, 201, 202) und das Ausflussloch (106) versetzt angeordnet sind.

8. Radiofrequenzablationskatheter gemäß Anspruch 6, wobei
die Außenwand der Elektrode (1) mit einer Aushöhlung (107) versehen ist, das Ausflussloch (106) in der Aushöhlung (107) angeordnet ist, der durchlässige Bereich die Aushöhlung (107) umgibt und der Zwischenraum zwischen der Innenwand der Infiltrationsabdeckung (20) und einer Oberfläche der Aushöhlung (107) definiert ist.

9. Radiofrequenzablationskatheter gemäß Anspruch 8, wobei
das Ausflussloch (106) aufgeweitet ist und der aufgeweitete Bereich als die Aushöhlung (107) dient; und
der Zwischenraum zwischen der Innenwand der Infiltrationsabdeckung (20) und der Oberfläche der Aushöhlung (107) mit zunehmendem Abstand vom Ausflussloch (106) abnimmt.

10. Radiofrequenzablationskatheter gemäß Anspruch 8, wobei
der Radiofrequenzablationskatheter ein zusätzliches Merkmal a), oder zusätzliche Merkmale a) und b) aufweist:
a) der Zwischenraum zwischen der Innenwand der Infiltrationsabdeckung (20) und der Oberfläche der Aushöhlung (107) vergrößert sich mit zunehmendem Abstand von dem Ausflussloch (106);
b) eine Aushöhlung (107) vorgesehen ist oder mehrere voneinander isolierte Aushöhlungen (107) vorgesehen sind, wobei in einer Aushöhlung (107) ein Ausflussloch (106) vorgesehen ist; und
wobei innerhalb einer Aushöhlung (107) der Zwischenraum zwischen der Innenwand der Infiltrationsabdeckung (20) und der Oberfläche der Aushöhlung (107) mit zunehmendem Abstand von dem Ausflussloch (106) in der Aushöhlung zunimmt.

11. Radiofrequenzablationskatheter gemäß Anspruch 8, wobei
der Radiofrequenzablationskatheter ein zusätzliches Merkmal c), oder zusätzliche Merkmale c) und d) aufweist:
c) die Aushöhlung (107) ist eine Verteilungsnut, die sich entlang der axialen Richtung der Elektrode (1) erstreckt, wobei
mehrere Gruppen von Ausflusslöchern (106) in einer Umfangsrichtung der Elektrode (1) angeordnet sind, und jede Gruppe von Ausflusslöchern (106) einer jeweiligen Verteilungsnut entspricht;
d) ein Ausflussloch (106) ist an einem Boden jeder Verteilungsnut definiert, und die Tiefe der Verteilungsnut nimmt mit zunehmendem Abstand vom Ausflussloch (106) zu.

12. Radiofrequenzablationskatheter gemäß Anspruch 1, wobei
der Durchmesser der Dispersionsöffnung (200, 201, 202) mit zunehmendem Abstand von dem Ausflussloch (106) zunimmt.

13. Radiofrequenzablationssystem für Lungen, umfassend:
einen Radiofrequenzablationskatheter gemäß einem der Ansprüche 1 bis 12;
eine Wärmeaustauschmedium-Zuführungsvorrichtung, die dazu eingerichtet ist, der Umgebung einer Elektrode (1) des Radiofrequenzablationskatheters ein Wärmeaustauschmedium zuzuführen; und
ein Steuermodul (140), das dazu eingerichtet ist, die Wärmeaustauschmedium-Zuführungsvorrichtung auf der Grundlage von Impedanzinformationen in einem Schaltkreis, in dem sich die Elektrode (1) des Radiofrequenzablationskatheters befindet, entsprechend zu steuern.

14. Radiofrequenzablationssystem für Lungen gemäß Anspruch 13, das ferner eine Temperaturdetektionsvorrichtung zum Erfassen von Temperaturinformationen in einem Umgebungsbereich der Elektrode (1) umfasst, wobei
das Steuermodul (140) ferner dazu eingerichtet ist, den Ablationsprozess gemäß den Temperaturinformationen zu veranlassen oder zu steuern.

15. Radiofrequenzablationssystem für Lungen gemäß Anspruch 14, wobei
eine oder mehrere Temperaturdetektionsvorrichtungen vorgesehen sind und ein Abstand von einer Position von mindestens einer der einen oder mehreren Temperaturdetektionsvorrichtungen zu der Elektrode (1) 0,5 cm bis 3 cm beträgt.

## Revendications

1. Cathéter d'ablation par radiofréquence, comprenant une électrode (1), dans lequel un canal d'écoulement de milieu d'échange de chaleur est défini à l'intérieur de l'électrode (1) ; l'électrode (1) est munie d'un dispositif de dispersion ; au moins un trou de dispersion (200) est ménagé sur le dispositif de dispersion et communique avec le canal d'écoulement de milieu d'échange de chaleur ; et un milieu d'échange de chaleur délivré en sortie du canal d'écoulement de milieu d'échange de chaleur est distribué par le dispositif de dispersion et s'écoule en sortie par celui-ci ;
dans lequel une pluralité de trous de dispersion (200) sont ménagés pour la formation d'une couche protectrice de milieu d'échange de chaleur uniforme sur une périphérie externe de l'électrode (1) ;
**caractérisé en ce que** le dispositif de dispersion et l'électrode (1) sont formés séparément, un trou d'écoulement de sortie (106) communiquant avec le canal d'écoulement de milieu d'échange de chaleur est défini sur une paroi externe de l'électrode (1), le dispositif de dispersion est une enveloppe d'infiltration (20) montée sur l'électrode (1) en encerclant le trou d'écoulement de sortie (106), un espace est défini entre une paroi périphérique interne de l'enveloppe d'infiltration (20) et la paroi périphérique externe de l'électrode (1), et les trous de dispersion (200) sont définis sur l'enveloppe d'infiltration (20), et dans lequel le milieu d'échange de chaleur délivré en sortie du trou d'écoulement de sortie (106) s'écoule vers l'espace entre l'électrode (1) et l'enveloppe d'infiltration (20), et s'écoule ensuite en sortie par les trous de dispersion (200), en formant une couche d'eau sur une surface externe de l'enveloppe d'infiltration (20).

2. Cathéter d'ablation par radiofréquence selon la revendication 1, dans lequel le canal d'écoulement de milieu d'échange de chaleur comprend un canal d'écoulement principal (115) et une pluralité de canaux d'écoulement de ramification (116) communiquant avec le canal d'écoulement principal (115), et dans lequel une extrémité de chaque canal d'écoulement de ramification (116) s'étend jusqu'à une surface externe de l'électrode (1).

3. Cathéter d'ablation par radiofréquence selon la revendication 1, dans lequel l'enveloppe d'infiltration (20) est fixée à l'électrode (1), montée de manière rotative sur l'électrode (1) autour de l'axe de l'électrode (1), ou montée de manière coulissante sur l'électrode (1) le long de la direction axiale de l'électrode (1).

4. Cathéter d'ablation par radiofréquence selon la revendication 1, dans lequel une pluralité d'enveloppes d'infiltration (20) sont montées sur l'électrode (1), et chaque enveloppe d'infiltration (20) se déplace indépendamment par rapport à l'électrode (1) ou au moins deux enveloppes d'infiltration (20) se déplacent en association les unes avec les autres.

5. Cathéter d'ablation par radiofréquence selon la revendication 1, dans lequel l'enveloppe d'infiltration (20) est d'une structure cylindrique qui est fermée de manière circonférentielle, et est montée autour de la périphérie externe de l'électrode (1), dans lequel le nombre des trous de dispersion (200) est supérieur au nombre de trou d'écoulement de sortie (106).

6. Cathéter d'ablation par radiofréquence selon la revendication 1, dans lequel au moins une partie de l'enveloppe d'infiltration (20) se présente sous la forme d'une zone perméable où les trous de dispersion (200) sont répartis, et une zone de l'électrode (1) où le trou d'écoulement de sortie (106) est défini correspond à la zone perméable, l'espace étant défini entre la zone et une paroi interne de la zone perméable.

7. Cathéter d'ablation par radiofréquence selon la revendication 6, dans lequel les trous de dispersion (200, 201, 202) et le trou d'écoulement de sortie (106) sont agencés d'une manière décalée.

8. Cathéter d'ablation par radiofréquence selon la revendication 6, dans lequel la paroi externe de l'électrode (1) est munie d'une zone en retrait (107), le trou d'écoulement de sortie (106) est agencé dans la zone en retrait (107), la zone perméable encercle la zone en retrait (107), et l'espace est défini entre la paroi interne de l'enveloppe d'infiltration (20) et une surface de la zone en retrait (107).

9. Cathéter d'ablation par radiofréquence selon la revendication 8, dans lequel le trou d'écoulement de sortie (106) est évasé, et la zone évasée constitue la zone en retrait (107) ; et l'espace entre la paroi interne de l'enveloppe d'infiltration (20) et la surface de la zone en retrait (107) diminue à mesure que la distance par rapport au trou d'écoulement de sortie (106) augmente.

10. Cathéter d'ablation par radiofréquence selon la revendication 8, dans lequel le cathéter d'ablation par radiofréquence possède une caractéristique supplémentaire a), ou des caractéristiques supplémentaires a) et b) :
a) l'espace entre la paroi interne de l'enveloppe d'infiltration (20) et la surface de la zone en retrait (107) augmente à mesure que la distance par rapport au trou d'écoulement de sortie (106) augmente ;
b) une zone en retrait (107) est ménagée ou plusieurs zones en retrait (107) isolées les unes des autres sont ménagées, un trou d'écoulement de sortie (106) étant ménagé dans une zone en retrait (107) ; et dans lequel au sein d'une zone en retrait (107), l'espace entre la paroi interne de l'enveloppe d'infiltration (20) et la surface de la zone en retrait (107) augmente à mesure que la distance par rapport au trou d'écoulement de sortie (106) dans la zone en retrait augmente.

11. Cathéter d'ablation par radiofréquence selon la revendication 8, dans lequel le cathéter d'ablation par radiofréquence possède une caractéristique supplémentaire c), ou des caractéristiques supplémentaires c) et d) :
c) la zone évidée (107) est une rainure de distribution s'étendant le long de la direction axiale de l'électrode (1), plusieurs groupes de trous d'écoulement de sortie (106) sont agencés dans une direction circonférentielle de l'électrode (1), et chaque groupe de trous d'écoulement de sortie (106) correspond à une rainure de distribution respective ;
d) un trou d'écoulement de sortie (106) est défini au niveau d'un fond de chaque rainure de distribution, et la profondeur de la rainure de distribution augmente à mesure que la distance par rapport au trou d'écoulement de sortie (106) augmente.

12. Cathéter d'ablation par radiofréquence selon la revendication 1, dans lequel le diamètre du trou de dispersion (200, 201, 202) augmente à mesure que la distance par rapport au trou d'écoulement de sortie (106) augmente.

13. Système d'ablation par radiofréquence pour les poumons, comprenant :
un cathéter d'ablation par radiofréquence selon l'une quelconque des revendications 1 à 12 ;
un appareil d'administration de milieu d'échange de chaleur, configuré pour fournir un milieu d'échange de chaleur aux environs d'une électrode (1) du cathéter d'ablation par radiofréquence ; et
un module de commande (140), configuré pour piloter l'appareil d'administration de milieu d'échange de chaleur en conséquence sur la base d'informations d'impédance dans un circuit où l'électrode (1) du cathéter d'ablation par radiofréquence est située.

14. Système d'ablation par radiofréquence pour les poumons selon la revendication 13, comprenant en outre un dispositif de détection de température pour l'acquisition d'informations de température dans une zone environnante de l'électrode (1), dans lequel le module de commande (140) est outre configuré pour susciter ou commander le processus d'ablation selon les informations de température.

15. Système d'ablation par radiofréquence pour les poumons selon la revendication 14, dans lequel un ou plusieurs dispositifs de détection de température sont prévus, et une distance, d'une position d'au moins l'un parmi les uns ou plusieurs dispositifs de détection de température à l'électrode (1), est de 0,5 cm à 3 cm.
